(19) **Europäisches Patentamt**
**European Patent Office**
**Office européen des brevets**

(11) **EP 3 123 193 B1**

(12) **EUROPÄISCHE PATENTSCHRIFT**

(45) Veröffentlichungstag und Bekanntmachung des Hinweises auf die Patenterteilung:
**16.07.2025 Patentblatt 2025/29**

(21) Anmeldenummer: **15711794.6**

(22) Anmeldetag: **25.03.2015**

(51) Internationale Patentklassifikation (IPC):
**G01R 33/563** (2006.01) **A61B 5/055** (2006.01)

(52) Gemeinsame Patentklassifikation (CPC):
**G01R 33/56366; A61B 5/0263; A61B 5/055**

(86) Internationale Anmeldenummer:
**PCT/EP2015/056419**

(87) Internationale Veröffentlichungsnummer:
**WO 2015/144768 (01.10.2015 Gazette 2015/39)**

(54) **ASL-MRI UNTER VERWENDUNG EINER FOLGE VON MAGNETISCH MARKIERTEN ODER NICHT-MARKIERTEN FLUIDBOLI**

ASL MRI USING A SEQUENCE OF MAGNETICALLY LABELED OR UNLABELED FLUIDBOLI

IRM D'ASL UTILISANT UNE SÉRIE DE BOLUS FLUIDIQUES MARQUÉS MAGNÉTIQUEMENT OU NON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.03.2014 DE 102014205789**

(43) Veröffentlichungstag der Anmeldung:
**01.02.2017 Patentblatt 2017/05**

(73) Patentinhaber: **Fraunhofer-Gesellschaft zur Förderung**
**der angewandten Forschung e.V.**
**80686 München (DE)**

(72) Erfinder:
• **SAMSON-HIMMELSTJERNA, Federico von**
**81373 München (DE)**
• **GÜNTHER, Matthias**
**28359 Bremen (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**WO-A2-2007/035824      US-A1- 2012 293 171**

• **VON SAMSON-HIMMELSTJERNA F ET AL: "Time efficient and robust perfusion measurement using Walsh-reordered time encoded pCASL", PROCEEDINGS OF THE INTERNATIONAL SOCIETY FOR MAGNETIC RESONANCE IN MEDICINE, ISMRM, JOINT ANNUAL MEETING ISMRM-ESMRMB, MILAN, ITALY, 10-16 MAY 2014, no. 719, 25 April 2014 (2014-04-25), pages 719, XP040661799**
• **WOUTER M. TEEUWISSE ET AL: "Time-encoded pseudocontinuous arterial spin labeling: Basic properties and timing strategies for human applications", MAGNETIC RESONANCE IN MEDICINE, vol. 72, no. 6, 6 January 2014 (2014-01-06), pages 1712 - 1722, XP055193384, ISSN: 0740-3194, DOI: 10.1002/mrm.25083**
• **JACK A. WELLS ET AL: "In vivo hadamard encoded continuous arterial spin labeling (H-CASL)", MAGNETIC RESONANCE IN MEDICINE, vol. 63, no. 4, 1 April 2010 (2010-04-01), pages 1111 - 1118, XP055143323, ISSN: 0740-3194, DOI: 10.1002/mrm.22266**

**Beschreibung**

Die Erfindung bezieht sich auf ein System zum Auswerten von diagnostisch verwendbaren MR-Daten eines strömenden Fluids, ein Verfahren zum Auswerten von diagnostisch verwendbaren MR-Daten eines strömenden Fluids und ein Computerprogramm zum Auswerten von diagnostisch verwendbaren MR-Daten eines strömenden Fluids. Das Fluid ist bevorzugt Blut.

In der US-Patentschrift US 8,260,396 B2 (Guenther 2012) ist ein MR-System offenbart, das in vivo Blut innerhalb vorher bestimmbarer räumlicher Abschnitte der Gefäße, z.B. im Bereich der Halsschlagader, magnetisch markiert. Dies geschieht dort mittels eines arteriellen MR-Spin-Markierungsprozesses, auch arterial spin labeling (ASL) genannt. Diese Blutabschnitte werden Boli (sing. Bolus) genannt und stellen innerhalb eines Gefäßes räumlich definierte Bereiche von entweder markiertem oder nicht markiertem Blut dar. Letztere dienen bekanntlich als Kontroll- oder auch Vergleichsabschnitte und werden häufig auch so genannt. Bei dem in (Guenther 2012) beschriebenen MR-System, werden die Blutabschnitte zu nacheinander liegenden ersten Zeitpunkten magnetisch markiert oder nicht markiert, so dass eine strömende Folge von Label- und Kontrollboli entsteht. Mittels unterschiedlicher Kombinationen von Label-und Kontrollboli werden unterschiedliche Bolifolgen erzeugt. Technisch funktionell stellen die unterschiedlichen Folgen von Label-/Kontrollboli unterschiedliche Kodierungen des Blutes dar. Zur Kodierung wir hier bevorzugt eine Hadamardmatrix herangezogen, deren Elemente bestimmen, ob ein Label- oder ein Kontrollbolus erzeugt wird, und wobei jede Zeile der Matrix eine Folge von Label- und/oder Kontrollboli bestimmt. Jede Zeile der Matrix wird somit einer MR-Messung erster MR-Daten zugrunde gelegt. Für jede dieser Blutbolifolgen werden nach einer Wartezeit (oft auch inflow time (TI) genannt) in einem zweiten Bereich erste MR-Bilder, die auch Zwischenbilder genannt werden, bestimmt. Dieser zweite Bereich ist der Bereich zu dem die Blutbolifolge strömt, bei Guenther zum Beispiel das Gehirn. Diese ersten MR-Bilder werden dann im Dekodierschritt auf bekannte Weise zusammengefügt, insbesondere miteinander addiert oder voneinander subtrahiert. Zur Dekodierung wird bevorzugt die zur Kodierung verwendete Hadamardmatrix erst invertiert und dann im eigentlichen Dekodierungsschritt zur Bestimmung der mathematischen Operationen verwendet, die zur Zusammenfügung notwendig sind. Insbesondere werden dazu die zweiten MR-Daten je nach Eintrag einer Zeile der Matrix miteinander addiert oder voneinander subtrahiert. Auf diese Art und Weise werden zweite MR-Bilder dekodiert, die jeweils für einen zu einem bestimmten ersten Zeitpunkt erzeugten und zum zweiten Bereich geströmten einzelnen Blutbolus indikativ sind. Diese entsprechen jeweils einem ersten MR-Bild, das bestimmt worden wäre, wenn zum ersten Zeitpunkt nur ein einzelner ausgewählter markierter Blutbolus erzeugt worden wäre. Die verschiedenen zweiten MR-Bilder entsprechen dabei weiterhin verschiedenen TIs. Das von Guenther 2012 beschriebene MR-System ist sehr effizient, da sich mit ihm in vergleichsweise kurzer Zeit und ohne Verschlechterung des Signal-Rausch-Verhältnisses klinisch relevante MR-Daten über Organperfusion und/oder -durchblutung gewinnen lassen. Bei einer beispielhaften Umsetzung von (Guenther 2012) können aus N gemessenen MR-Bildern N-1 rekonstruierte MR-Bilder erzeugt werden und zur Erzeugung jedes dieser N-1 rekonstruierten MR-Bilder werden alle N gemessenen MR-Bilder benötigt. Mit den N-1 rekonstruierten MR-Bildern kann ein MR-Signal zu verschiedenen Wartezeiten TI bestimmt und somit voxel-/pixelweise der Signalverlauf über die Zeit ermittelt werden. Aus diesen Signal-Zeit-Kurven lassen sich dann voxel-/pixelweise verschiedene, für die klinische Diagnose relevante Parameter bestimmen.

Der Artikel "Time-Encoded pseudoContinuous Arterial Spin Labeling: Basic Properties and Timing Strategies for Human Applications" von W. Teeuwisse et al., Magnetic Resonance in Medicine, Band 72, Seiten 1712 bis 1722 (2014) offenbart ein zeitlich-kodiertes pseudokontinuierliches ASL-Verfahren (pCASL-Verfahren), bei dem beispielsweise die Dauer eines Blockes variabel ist, um einen T1-Zerfall zu kompensieren, oder bei dem die Nachmarkierungs-Verzögerungszeit (engl. "postlabeling delay time") verwendet wird, um eine arterielle Transitzeit (engl. "arterial transit time", ATT) zu erfassen.

Es ist eine Aufgabe der vorliegenden Erfindung, ein System, ein Verfahren und ein dazugehöriges Computerprogramm bereitzustellen, die es ermöglichen, die Erzeugung von MR-Daten eines strömenden Fluids zu verbessern.

Die Aufgabe wird durch ein System zum Auswerten von diagnostisch verwendbaren MR-Daten eines strömenden Fluids gemäß Anspruch 1 gelöst, wobei das System aufweist:

- eine Datenerfassungseinheit, die angepasst ist, mehrere MR-Messungen durchzuführen und dabei in jeder MR-Messung erste MR-Daten misst, indem in jeder Magnetresonanzmessung

    a) zu verschiedenen ersten Zeitpunkten in einem ersten Bereich das Fluid entweder magnetisch markiert oder nicht markiert wird, derart, dass eine strömende Folge von markierten und/oder nicht-markierten Fluidboli entsteht, und

    b) zu einem zweiten Zeitpunkt in einem zweiten Bereich, zu dem das Fluid ausgehend von dem ersten Bereich strömt, die ersten MR-Daten, die indikativ sind für die erzeugte Folge von markierten und/oder nicht-markierten Fluidboli, gemessen werden,

wobei die Datenerfassungseinheit angepasst ist, zu jeder Folge von markierten und/oder nicht-markierten Fluidboli eine komplementäre Folge von markierten und/oder nicht-markierten Fluidboli zu erzeugen, wobei die Magnetresonanzmessungen der ersten Magnetresonanzdaten von zueinander komplementären Folgen unmittelbar nacheinander durchgeführt werden, und

die so gemessenen ersten Magnetresonanzdaten bereitzustellen, oder

- eine Datenbereitstellungseinheit, die angepasst ist, gemäß a) und b) gemessene erste Magnetresonanzdaten bereitzustellen, und

- eine Matrixbereitstellungseinheit zum Bereitstellen einer zweiwertigen Bestimmungsmatrix, wobei eine Zeile der Bestimmungsmatrix eine Folge von markierten und/oder nicht-markierten Fluidboli repräsentiert und unterschiedliche Zeilen der Bestimmungsmatrix unterschiedlichen Magnetresonanzmessungen der ersten Magnetresonanz-Daten zugrunde gelegt werden, wobei die Bestimmungsmatrix durch eine Kombination einer Walsh-sortierten Hadamard-Matrix mit einer komplementären Walsh-sortierten Hadamard-Matrix gebildet ist, wobei die gebildete Matrix wechselweise die Zeilen der Walsh-sortierten Hadamard-Matrix und der komplementären Walsh-sortierten Hadamard-Matrix aufweist,

- eine Auswerteeinheit, die angepasst ist, basierend auf einer Zusammenfügung der in den verschiedenen MR-Messungen gemessenen ersten MR-Daten unter Berücksichtigung der Bestimmungsmatrix,

i) zweite Einzelfluidbolus-Magnetresonanzdaten zu bestimmen, die jeweils für einen zu einem bestimmten ersten Zeitpunkt erzeugten und zum zweiten Bereich geströmten einzelnen Fluidbolus indikativ sind, basierend auf einer Zusammenfügung der in den verschiedenen Magnetresonanzmessungen gemessenen ersten Magnetresonanzdaten, und

ii) zweite Kombinationsfluidbolus-MR-Daten zu bestimmen, die jeweils für einen Kombinations-Fluidbolus und einen zeitlichen Abstand zwischen einem der ersten Zeitpunkte und dem zweiten Zeitpunkt indikativ sind, wobei der Kombinations-Fluidbolus aus mehreren zu verschiedenen ersten Zeitpunkten markierten/nicht-markierten und zum zweiten Bereich geströmten Fluidboli (L, C) zusammengesetzt ist, wobei

- die Datenerfassungseinheit angepasst ist, die ersten Magnetresonanzdaten in einer Reihenfolge zu messen, die es ermöglicht, dass, bereits bevor sämtliche erste Magnetresonanzdaten gemessen worden sind, die zur Bestimmung der zweiten Einzelfluidbolus-Magnetresonanzdaten benötigt werden, in der Auswerteeinheit zweite Kombinationsfluidbolus-Magnetresonanzdaten bestimmt werden können, dadurch gekennzeichnet, dass die Walsh-sortierte Hadamard-Matrix und die komplementäre Walsh-sortierte Hadamard-Matrix jeweils einen Grad $2^k$ mit $k \geq 2$ aufweisen, und

- die Auswerteeinheit angepasst ist, die ersten Magnetresonanzdaten in einer Reihenfolge auszuwerten, die es ermöglicht, dass, bereits bevor sämtliche erste Magnetresonanzdaten gemessen worden sind, die zur Bestimmung der zweiten Einzelfluidbolus-Magnetresonanzdaten benötigt werden, zweite Kombinationsfluidbolus-Magnetresonanzdaten bestimmt werden können, wobei für die Bestimmung von zweiten Magnetresonanzdaten für einen Kombinationsfluidbolus weniger erste Magnetresonanz-daten verwendet werden als für die Bestimmung von zweiten Magnetresonanzdaten für einen Einzelfluidbolus.

Die Markierung/Nicht-Markierung gemäß dem oben genannten Merkmal a) kann auch als Kodierung des Fluids betrachtet werden - wie schon bei Guenther 2012. Die voranstehende Zusammenfügung, die auch als eine Kombination bezeichnet werden könnte, besteht bekanntlich (vgl. Guenther 2012) insbesondere aus mathematischen Operationen wie Addition und Subtraktion der ersten MR-Daten.

Durch Verwendung des oder der Kombinations-Fluidbolus/i wird die später noch näher erläuterte Möglichkeit geschaffen, Informationen über das Strömungsverhalten des Fluids zu erhalten, bevor sämtliche erste MR-Daten gemessen worden sind. Es ist daher beispielsweise nicht notwendig, einen gesamten Messprozess abzuwarten, und erst dann die zweiten MR-Daten zu bestimmen, wie es etwa in der einleitend genannten Druckschrift Guenther 2012 beschrieben ist. Insbesondere in diesem Sinne kann das System, das auch als MR-System bezeichnet werden kann, die Erzeugung von MR-Daten eines strömenden Fluids verbessern.

Im Ergebnis entsteht durch die Zusammensetzung des Kombinations-Fluidbolus aus zwei oder mehreren zusammenhängend markierten/nicht-markierten einzelnen Fluidboli effektiv ein vergleichsweise längerer Bolus, der vorstehend Kombinations-Fluidbolus genannt wurde. Ein Kombinations-Fluidbolus kann also auch als eine Aneinanderreihung von zwei oder mehr einzelnen Fluidboli verstanden werden.

Der Detailreichtum der zweiten MR-Daten nimmt zwar mit weniger und länger werdenden Fluidboli, d.h. im Endeffekt größeren zeitlichen Abtastschritten, ab. Dafür ergibt sich aber die Möglichkeit die zweiten MR-Daten aus weniger ersten MR-Daten zu erzeugen und somit auch schon frühzeitiger relevante zweite MR-Daten zu gewinnen, etwa klinisch relevante Aussagen zur Organperfusion. Diese Informationen können vorteilhaft auch bei der weiteren Steuerung der MR-Messung berücksichtigt werden.

Das strömende Fluid ist bevorzugt Blut. Das strömende Fluid kann aber auch ein anderer flüssiger oder gasförmiger Stoff sein; beispielsweise auch eine andere Körperflüssigkeit. Die ersten MR-Daten sind bevorzugt zweidimensionale oder dreidimensionale MR-Bilder bestehend aus Pixeln oder Voxeln.

Erfindungsgemäß ist die Auswerteeinheit derart angepasst, dass zum Bestimmen derjenigen zweiten MR-Daten, die jeweils für einen Kombinations-Fluidbolus und einen zeitlichen Abstand zwischen einem der ersten Zeitpunkte und einem der zweiten Zeitpunkte indikativ sind, weniger erste MR-Daten verwendet werden als für die Bestimmung derjenigen zweiten MR-Daten, die jeweils für einen einzelnen Fluidbolus indikativ sind. Das heißt, dass, wenn beispielsweise einige erste MR-Daten fehlerhaft sein sollten, zum Beispiel weil sich ein Patient bewegt hat, so dass deshalb zweite MR-Daten, die jeweils für einen einzelnen Fluidbolus indikativ sind, nicht bestimmt werden können, dennoch zweite MR-Daten bestimmt werden könnten, die jeweils für einen Kombinations-Fluidbolus und einen zeitlichen Abstand zwischen einem der ersten Zeitpunkte und dem zweiten Zeitpunkt indikativ sind. Zudem könnten grundsätzlich, bereits bevor sämtliche erste MR-Daten gemessen worden sind, die zur Bestimmung der zweiten MR-Daten benötigt werden, die jeweils für einen einzelnen Fluidbolus indikativ sind, zweite MR-Daten bestimmt werden, die jeweils für einen Kombinations-Fluidbolus und einen zeitlichen Abstand zwischen einem der ersten Zeitpunkte und dem zweiten Zeitpunkt indikativ sind. Es ist beispielsweise denkbar, dass in Echtzeit bereits während der Messung diejenigen zweiten MR-Daten bestimmt werden, die jeweils für einen Kombinations-Fluidbolus und einen zeitlichen Abstand zwischen einem der ersten Zeitpunkte und dem zweiten Zeitpunkt indikativ sind.

Das MR-System weist erfindungsgemäß eine Matrixbereitstellungseinheit zum Bereitstellen einer zweiwertigen Bestimmungsmatrix auf, d.h. einer Matrix deren Elemente einen von zwei möglichen Werten haben. Dabei repräsentiert eine Zeile der Bestimmungsmatrix eine Folge von markierten- und/oder nicht-markierten Fluidboli, wobei unterschiedliche Zeilen der Bestimmungsmatrix unterschiedlichen MR-Messungen der ersten MR-Daten zu Grunde gelegt werden. Die Bestimmungsmatrix kann auch als Matrix aufgefasst werden, welche die Kodierung der Fluidboli bestimmt. Die Auswerteeinheit ist bevorzugt angepasst, a) einen Vektor zu bilden, wobei ein Vektorelement durch einen MR-Wert der ersten MR-Daten gebildet wird und verschiedene Vektorelemente verschiedenen MR-Messungen der ersten MR-Daten entsprechen, b) die Bestimmungsmatrix zu invertieren, und c) die zweiten MR-Daten zu bestimmen, indem die invertierte Bestimmungsmatrix auf den Vektor angewendet wird, insbesondere mit ihm multipliziert wird. Die Einträge einer zweiwertigen Bestimmungsmatrix weisen lediglich zwei Werte auf, beispielsweise +1 und -1, welche markierte und nicht-markierte Fluidboli repräsentieren. Die Bestimmungsmatrix umfasst also mehrere Matrixelemente, wobei jedes dieser Matrixelemente einen von zwei möglichen Werten aufweisen kann. Der Ausdruck "zweiwertig" bezieht sich demnach auf die Anzahl möglicher Werte eines Matrixelements. Er bezieht sich aber beispielsweise nicht auf die Anzahl der Matrixelemente.

Ein erster MR-Wert, das heißt ein MR-Wert der ersten MR-Daten, der ein Vektorelement bildet, ist bevorzugt ein Bildwert, insbesondere ein Voxelwert oder ein Pixelwert, eines ersten MR-Bildes. Das heißt, die Bestimmung der zweiten MR-Daten kann Pixel für Pixel bzw. Voxel für Voxel erfolgen, wobei für jeden Pixel bzw. für jeden Voxel der unterschiedlichen ersten MR-Bilder, die in diesem Beispiel die ersten MR-Daten darstellen, ein Vektor gebildet wird. Beispielsweise wird für den Pixel an der Stelle (1,1) ein Vektor gebildet, der die entsprechenden Bildwerte an den Stellen (1,1) der zu den unterschiedlichen ersten Zeitpunkten aufgenommenen ersten MR-Bilder aufweist.

In einer Ausführungsform werden demnach im Dekodierschritt (ein solcher Schritt ist grundsätzlich bereits aus Guenther 2012 bekannt) als erste MR-Daten zum Beispiel erste MR-Bilder zusammengefügt, insbesondere addiert und/oder subtrahiert, um als zweite MR-Daten zweite MR-Bilder zu erzeugen. Die Erzeugung eines zweiten MR-Bildes durch Zusammenfügung mehrerer erster MR-Bilder kann in einer Ausführungsform dadurch erfolgen, dass die ersten MR-Bilder gemäß den Einträgen jeweils einer Zeile der invertierten Bestimmungsmatrix zu einem zweiten MR-Bild aufaddiert und/oder -subtrahiert werden. Die invertierte Bestimmungsmatrix kann hier auch als Dekodiermatrix aufgefasst werden. Rechnerisch kann das zum Beispiel wie folgt realisiert werden: Es wird ein Vektor gebildet, bei dem jeder Vektoreintrag genau einem ersten MR-Bild zugeordnet ist und dem Voxelwert an genau einer vorher festgelegten Voxelstelle des jeweiligen ersten MR-Bildes entspricht. Die invertierte Bestimmungsmatrix wird dann mit diesem Vektor multipliziert. Das Ergebnis ist ein zweiter Vektor, bei dem jeder Vektoreintrag wiederum genau einem zweiten MR-Bild zugeordnet ist und dem Voxelwert an der derselben, vorher festgelegten Voxelstelle des jeweiligen zweiten MR-Bildes entspricht. Dieses Verfahren wird in beschriebener Ausführungsform für jeden Voxel der ersten und zweiten MR-Bilder durchgeführt. So kann beispielsweise, um einen Voxelwert für die Voxelstelle (1,1,1) eines zweiten MR-Bildes zu bestimmen, eine erste Zeile der invertierten Bestimmungsmatrix mit einem Vektor multipliziert werden, der die in den unterschiedlichen ersten MR-Bildern an der Voxelstelle (1,1,1) enthaltenen Voxelwerte aufweist. Um für dieses zweite MR-Bild den Voxelwert zu ermitteln, der an der Stelle (1,1,2) angeordnet ist, kann dieselbe erste Zeile der invertierten Bestimmungsmatrix mit einem Vektor multipliziert werden, der die Voxelwerte der ersten MR-Bilder an der Voxelstelle (1,1,2) aufweist. Dies wird für alle Voxel durchgeführt, so dass die Voxelwerte aller Voxelpositionen für dieses zweite MR-Bild auf diese Art und Weise bestimmt sind. Verschiedene Zeilen der invertierten Bestimmungsmatrix entsprechen dann jeweils genau einem zweiten MR-Bild.

Erfindungsgemäß ist die Datenerfassungseinheit des Weiteren angepasst, zu jeder Folge von markierten und/oder nicht-markierten Fluidboli eine hierzu komplementäre Folge zu erzeugen, wobei eine erste Folge komplementär zu einer zweiten Folge ist, wenn an jeder Stelle der ersten Folge, die einer Stelle der zweiten Folge entspricht, an der ein markierter Fluidbolus angeordnet ist, ein nicht-markierter Fluidbolus angeordnet ist, und umgekehrt. In einer Ausführungsform wird für jede strömende Folge von markierten und/oder nicht-markierten Fluidboli ein erstes MR-Bild erzeugt. Da bevorzugt zu jeder Folge von markierten und/oder nicht-markierten Fluidboli eine komplementäre Folge vorhanden ist, wird zusätzlich für jedes erste MR-Bild bevorzugt ein weiteres erstes MR-Bild erzeugt, das auf Basis einer strömenden Folge von markierten und/oder nicht-markierten Fluidboli erzeugt worden ist, die komplementär zur strömenden Folge von markierten und/oder nicht-markierten Fluidboli ist, auf der das vorangegangene erste MR-Bild basiert. In einer bevorzugten Ausführungsform gibt es demnach zu jedem ersten MR-Bild, das auf Basis einer ersten strömenden Folge erzeugt worden ist, ein weiteres erstes MR-Bild, das auf Basis einer zur ersten komplementären strömenden Folge erzeugt worden ist.

Die Bereitstellung von zueinander komplementären Folgen von markierten/nicht-markierten Fluidboli hat gegenüber der eingangs genannten Druckschrift Guenther 2012 zum Beispiel folgenden Vorteil: Dort können aus N ersten MR-Daten nur N-1 zweite MR-Daten bestimmt werden, beispielsweise können zweite MR-Daten, die für den ersten Fluidbolus indikativ sind, nicht erzeugt werden. Verwendet man jedoch die Informationen aus den beiden zueinander komplementären Fluidboli-Folgen können zweite MR-Einzeldaten, die für alle N Fluidboli indikativ sind, erzeugt werden. Weiter können dann auch zweite MR-Kombinationsdaten, die für Kombinations-Fluidboli, die den ersten Fluidbolus enthalten, indikativ sind, aus den ersten MR-Daten erzeugt werden

Die Datenerfassungseinheit ist erfindungsgemäß angepasst, MR-Messungen der ersten MR-Daten, die zueinander komplementäre Folgen aufweisen, unmittelbar nacheinander durchzuführen. Das heißt, dass nach einer ersten MR-Messung mit einer ersten Fluidboli-Folge eine zweite derartige MR-Messung mit einer zweiten Folge durchgeführt wird, die zu der ersten Folge komplementär ist, wonach dritte und vierte MR-Messungen von ersten MR-Daten mit dritten und vierten Folgen durchgeführt werden, wobei die vierte Folge komplementär ist zu der dritten Folge, und so weiter. Die Datenerfassungseinheit kann demnach beispielsweise so programmiert sein, dass sie zunächst ein erstes MR-Bild erzeugt, das auf einer ersten strömenden Folge von markierten und/oder nicht-markierten Fluidboli basiert, wonach ein weiteres erstes MR-Bild erzeugt wird, das auf einer zweiten Folge von markierten und/oder nicht-markierten Fluidboli basiert, die zu der ersten Folge komplementär ist. Danach kann ein weiteres erstes MR-Bild mittels der entsprechend programmierten Datenerfassungseinheit erzeugt werden, das auf einer dritten Folge von markierten und/oder nicht-markierten Fluidboli basiert, wonach ein weiteres erstes MR-Bild erzeugt werden kann, das auf einer vierten Folge von markierten und/oder nicht-markierten Fluidboli basiert, die zu der dritten Folge von markierten und/oder nicht-markierten Fluidboli komplementär ist, und so weiter.

In einer Ausführungsform repräsentieren die Zeilen der Walsh-sortierten Hadamard-Matrix eine erste Gruppe von Folgen markierter und/oder nicht-markierter Fluidboli, wobei verschiedene Zeilen der Walsh-sortierten Hadamard-Matrix verschiedenen MR-Messungen der ersten MR-Daten zu Grunde gelegt werden. Zudem repräsentieren die Zeilen der komplementären Walsh-sortierten Hadamard-Matrix eine zweite Gruppe von Folgen markierter und/oder nicht-markierter Fluidboli, die komplementär zu den Folgen der ersten Gruppe sind, und wobei verschiedene Zeilen der komplementären Walsh-sortierten Hadamard-Matrix verschiedenen MR-Messungen der ersten MR-Daten entsprechen. Außerdem ist die Auswerteeinheit angepasst, a) einen ersten Vektor zu bilden, wobei ein Vektorelement des ersten Vektors jeweils durch einen MR-Wert der ersten MR-Daten gebildet wird und verschiedene Vektorelemente des ersten Vektors verschiedenen MR-Messungen entsprechen, die wiederum verschiedenen Folgen der ersten Gruppe entsprechen, b) einen zweiten Vektor zu bilden, wobei ein Vektorelement des zweiten Vektors durch einen MR-Wert der ersten MR-Daten gebildet wird und verschiedene Vektorelemente des zweiten Vektors verschiedenen MR-Messungen entsprechen, die wiederum verschiedenen Folgen der zweiten Gruppe entsprechen, c) die Walsh-sortierte Hadamard-Matrix und die komplementäre Walsh-sortierte Hadamard-Matrix zu invertieren, und d) die zweiten MR-Daten zu bestimmen, indem die invertierte Walsh-sortierte Hadamard-Matrix auf den ersten Vektor und die invertierte komplementäre Walsh-sortierte Hadamard-Matrix auf den zweiten Vektor angewendet wird, insbesondere durch Multiplikation.

Erfindungsgemäß gibt es demnach eine erste Gruppe von Folgen markierter und/oder nicht-markierter Fluidboli, wobei für jede Folge dieser ersten Gruppe jeweils ein erstes MR-Bild erzeugt werden kann. Da die Zeilen der Walsh-sortierten Hadamard-Matrix die Folgen markierter und/oder nicht-markierter Fluidboli der ersten Gruppe repräsentieren und da eine jeweilige Folge markierter und/oder nicht-markierter Fluidboli eine MR-Messung charakterisiert, bei der vorzugsweise ein jeweiliges erstes MR-Bild erzeugt worden ist, entspricht eine einzelne Zeile der Walsh-sortierten Hadamard-Matrix einer einzelnen MR-Messung, die vorzugsweise zu einem einzelnen ersten MR-Bild geführt hat, und verschiedene Zeilen der Walsh-sortierten Hadamard-Matrix entsprechen verschiedenen MR-Messungen, die vorzugsweise zu verschiedenen ersten MR-Bildern geführt haben.

In einer Ausführungsform wird die Walsh-sortierte Hadamard-Matrix invertiert und die invertierte Walsh-sortierte Hadamard-Matrix wird mit einem ersten Vektor multipliziert, der die verschiedenen Voxelwerte der verschiedenen ersten MR-Bilder an derselben Voxelstelle (1,1,1) aufweist, um für zweite MR-Bilder Voxelwerte an derselben Voxelstelle (1,1,1) zu berechnen. Für die anderen Voxelstellen wird auch jeweils ein erster Vektor erzeugt und mit der invertierten Walsh-sortierten Hadamard-Matrix multipliziert, um Voxelwerte an den entsprechenden Voxelstellen für die zweiten MR-Bilder zu erzeugen. Auf diese Art wird für die erste Gruppe von Folgen markierter und/oder nicht-markierter Fluidboli eine erste Gruppe von zweiten MR-Bildern erzeugt. Auf die gleiche Art und Weise kann eine komplementäre Walsh-sortierte Hadamard-Matrix für eine zweite Gruppe von Folgen markierter und/oder nicht-markierter Fluidboli erzeugt und invertiert werden, wobei die invertierte komplementäre Walsh-sortierte Hadamard-Matrix mit entsprechenden zweiten Vektoren multipliziert werden kann, um auch für die zweite Gruppe von Folgen markierter und/oder nicht-markierter Fluidboli zweite MR-Daten zu erzeugen.

Erfindungsgemäß ist die Datenerfassungseinheit angepasst, MR-Messungen zeitlich nacheinander geordnet nach der Anzahl von Wechseln zwischen markierten und/oder nicht-markierten Fluidboli in der entsprechenden Folge durchzuführen. Die MR-Messungen sind zeitlich nacheinander gemäß einer Walsh-Sortierung geordnet. Das heißt dass die oben beschriebene Bestimmungsmatrix Zeilen aufweist, die gemäß der Walsh-Sortierung angeordnet sind. In einer Ausführungsform kann daher eine Datenerfassungseinheit, das heißt insbesondere ein entsprechendes MR-System, so eingestellt sein, dass zuerst ein erstes MR-Bild auf Basis einer Folge von markierten und/oder nicht-markierten Fluidboli erzeugt wird, die die geringste Anzahl von Wechseln zwischen markierten und/oder nicht-markierten Fluidboli aufweist. Als Nächstes könnte ein weiteres erstes MR-Bild auf Basis einer Folge erzeugt werden, die die zweitwenigsten Wechsel zwischen markierten und/oder nicht-markierten Fluidboli aufweist, und so weiter. Wenn mehrere Folgen dieselbe Anzahl von Wechseln zwischen markierten und/oder nicht-markierten Fluidboli aufweisen, werden die entsprechenden ersten MR-Bilder nacheinander erzeugt, bevor ein weiteres erstes MR-Bild erzeugt wird, das auf einer Folge basiert, die eine größere Anzahl von Wechseln zwischen markierten und/oder nicht-markierten Fluidboli aufweist.

In einer bevorzugten Ausführungsform ist die Auswerteeinheit angepasst, Additions- und/oder Subtraktionsdaten zu bestimmen, indem in verschiedenen MR-Messungen gemessene erste MR-Daten voneinander addiert und/oder subtrahiert werden, und die zweiten MR-Daten basierend auf den Additions- und/oder Subtraktionsdaten zu bestimmen. Das heißt beispielsweise, dass entsprechende Pixelwerte oder Voxelwerte von ersten MR-Bildern unterschiedlicher MR-Messungen addiert und/oder subtrahiert werden, um die zweiten MR-Daten zu bestimmen, die jeweils für einen - aus mehreren zu verschiedenen ersten Zeitpunkten erzeugten und zum zweiten Bereich geströmten Fluidboli zusammengesetzten - Kombinations-Fluidbolus und einen zeitlichen Abstand zwischen einem der ersten Zeitpunkte und dem zweiten Zeitpunkt indikativ sind.

Die oben genannte Aufgabe wird außerdem durch ein Verfahren zum Auswerten von diagnostisch verwendbaren MR-Daten eines strömenden Fluids gemäß Anspruch 4 gelöst.

Die oben genannte Aufgabe wird des Weiteren durch ein Computerprogramm zum Auswerten von MR-Daten eines strömenden Fluids gemäß Anspruch 6 gelöst.

Es sollte verstanden werden, dass das System nach Anspruch 1, das Verfahren nach Anspruch 4 und das oben genannte Computerprogramm ähnliche und/oder identische bevorzugte Ausführungsformen aufweisen, wie sie insbesondere in den abhängigen Ansprüchen definiert sind.

Es sollte zudem verstanden werden, dass eine bevorzugte Ausführungsform der vorliegenden Erfindung durch jede Kombination der abhängigen Ansprüche und/oder der in der vorliegenden Anmeldung genannten Ausführungsformen mit den jeweiligen unabhängigen Ansprüchen gebildet werden kann, sofern die jeweilige Kombination in den durch die Ansprüche definierten Schutzumfang der Erfindung fällt.

Ausführungsformen der Erfindung werden unter Bezugnahme auf folgende Figuren beschrieben, wobei

Fig. 1     schematisch und exemplarisch eine Ausführungsform eines MR-Systems zeigt,

Fig. 2     schematisch und exemplarisch verschiedene Folgen von ,markierten und nicht-markierten Fluidboli und zweite MR-Daten zeigt,

Fig. 3     schematisch und exemplarisch eine Ausführungsform eines Auswertesystems zum Auswerten von ersten MR-Daten zeigt und

Fig. 4     ein Flussdiagramm zeigt, das exemplarisch ein MR-Verfahren darstellt.

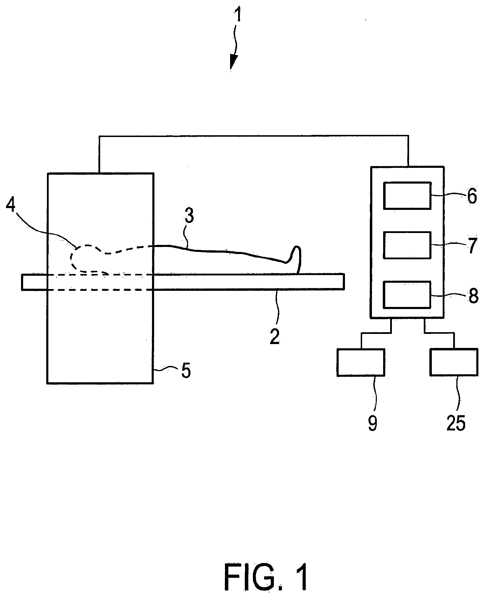

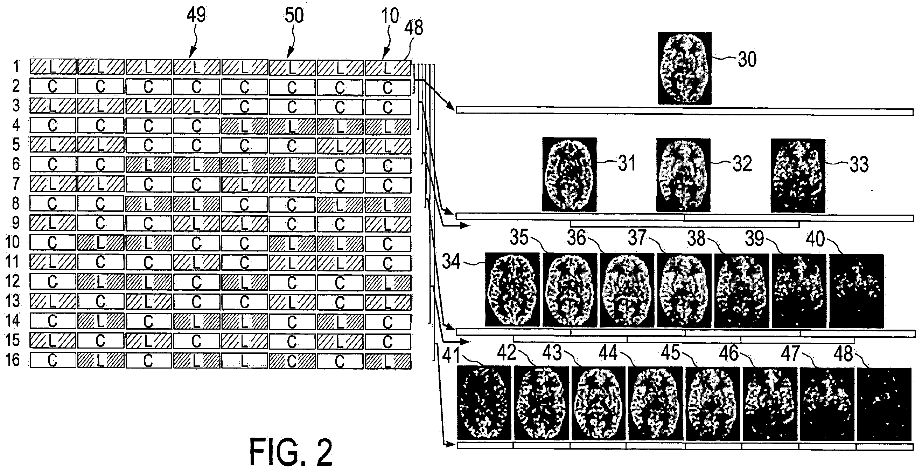

Fig. 1 zeigt schematisch und beispielhaft eine Ausführungsform eines MR-Systems. Das MR-System 1 umfasst eine Datenerfassungseinheit 5 zum Erfassen von ersten MR-Daten eines Patienten 3, der auf einer Patientenliege 2 angeordnet ist. In dieser Ausführungsform ist die Datenerfassungseinheit 5 ausgebildet, mehrere MR-Messungen im Kopfbereich 4 des Patienten 3 durchzuführen. In jeder MR-Messung werden erste MR-Daten gemessen, indem a) zu verschiedenen ersten Zeitpunkten das in einem oder mehreren Blutgefäßen, insbesondere Arterien, strömende Blut in einem ersten Gefäßabschnitt beim Patienten 3 entweder magnetisch markiert oder nicht markiert wird, um eine strömende Folge von markierten und/oder nicht-markierten Fluidboli, hier markierten und/oder nicht-markierten Blutboli (Fig. 2; L,C), zu erzeugen, und b) zu einem zweiten Zeitpunkt in einem zweiten Bereich, zu dem das Blut ausgehend von dem ersten Ort , d.h. dem vorgenannten Gefäßabschnitt, strömt, die ersten MR-Daten, die indikativ sind für die erzeugte Folge von markierten und/oder nicht-markierten Blutboli gemessen werden. Das MR-System 1 weist des Weiteren eine Auswerteeinheit 7 zum Auswerten der in den verschiedenen MR-Messungen gemessenen ersten MR-Daten auf, wobei die Auswerteeinheit 7 angepasst ist, zweite MR-Daten zu bestimmen, die jeweils für einen - aus mehreren zu verschiedenen ersten Zeitpunkten und zum zweiten Bereich geströmten Blutboli zusammengesetzten - Kombinations-Blutbolus und einen zeitlichen Abstand zwischen einem der ersten Zeitpunkte und dem zweiten Zeitpunkt indikativ sind. Die Bestimmung der zweiten MR-Daten basiert auf einer Zusammenfügung der in den verschiedenen MR-Messungen gemessenen ersten MR-Daten. Diese Zusammenfügung umfasst insbesondere ein Addieren und Subtrahieren erster MR-Daten, wobei insbesondere die Elemente einer Dekodierungsmatrix bestimmen, ob subtrahiert oder addiert wird.

Dies kann beispielsweise, analog zu Guenther 2012, auch beschrieben werden durch eine Multiplikation der Dekodiermatrix mit einem Vektor, dessen einzelne Einträge von einem Bildwert, z.B. dem Wert eines Voxels, von jeweils einer der ersten MR-Daten gebildet werden. Die Auswerteeinheit 7 ist des Weiteren angepasst, auch solche zweite MR-Daten zu bestimmen, die jeweils für einen zu einem bestimmten ersten Zeitpunkt erzeugten und zum zweiten Bereich geströmten einzelnen Blutbolus indikativ sind, wiederum basierend auf einer Zusammenfügung der in den verschiedenen MR-Messungen gemessenen ersten MR-Daten. In dieser Ausführungsform sind die ersten und zweiten MR-Daten MR-Bilder.

Das MR-System 1 weist des Weiteren eine Matrixbereitstellungseinheit 6 zum Bereitstellen einer zweiwertigen Bestimmungsmatrix auf, wobei jede Zeile der Bestimmungsmatrix durch eine Folge von Markierungs- und/oder Nichtmarkierungsbereichen gebildet wird. Diese Markierungs-/Nichtmarkierungsbereiche legen fest, ob das Blut markiert oder nicht markiert wird und sie entsprechen insoweit den markierten/ nicht-markierten Blutboli. Die Bestimmungsmatrix enthält also die Vorschrift für die Kodierung der einzelnen Blutboli. Die verschiedenen MR-Messungen der ersten MR-Daten entsprechen verschiedenen Zeilen der Bestimmungsmatrix, genauer verschiedenen Folgen von Blutboli, die gemäß der Bestimmungsmatrix kodiert wurden. Zudem ist die Auswerteeinheit 7 angepasst, einen Vektor zu bilden, wobei ein Vektorelement durch einen ersten MR-Wert gebildet wird, der in dieser Ausführungsform einem Bildwert entspricht, und verschiedene Vektorelemente verschiedenen MR-Messungen entsprechen. Die Auswerteeinheit 7 ist des Weiteren angepasst, die Bestimmungsmatrix zu invertieren - also aus ihr eine Dekodiermatrix zu machen - und die zweiten MR-Daten zu bestimmen, indem die invertierte Bestimmungsmatrix auf den Vektor angewendet wird.

In diesem Ausführungsbeispiel werden 16 MR-Messungen durchgeführt, wobei in jeder MR-Messung eine Folge von acht markierten- und/oder nicht-markierten Blutboli erzeugt wird. Die Kodierung der Folgen von Blutboli in den unterschiedlichen MR-Messungen kann in diesem Fall beispielsweise durch folgende Matrix M repräsentiert werden:

$$\mathbf{M} = \begin{pmatrix} +1 & +1 & +1 & +1 & +1 & +1 & +1 & +1 \\ -1 & -1 & -1 & -1 & -1 & -1 & -1 & -1 \\ +1 & +1 & +1 & +1 & -1 & -1 & -1 & -1 \\ -1 & -1 & -1 & -1 & +1 & +1 & +1 & +1 \\ +1 & +1 & -1 & -1 & -1 & -1 & +1 & +1 \\ -1 & -1 & +1 & +1 & +1 & +1 & -1 & -1 \\ +1 & +1 & -1 & -1 & +1 & +1 & -1 & -1 \\ -1 & -1 & +1 & +1 & -1 & -1 & +1 & +1 \\ +1 & -1 & -1 & +1 & +1 & -1 & -1 & +1 \\ -1 & +1 & +1 & -1 & -1 & +1 & +1 & -1 \\ +1 & -1 & -1 & +1 & -1 & +1 & +1 & -1 \\ -1 & +1 & +1 & -1 & +1 & -1 & -1 & +1 \\ +1 & -1 & +1 & -1 & -1 & +1 & -1 & +1 \\ -1 & +1 & -1 & +1 & +1 & -1 & +1 & -1 \\ +1 & -1 & +1 & -1 & +1 & -1 & +1 & -1 \\ -1 & +1 & -1 & +1 & -1 & +1 & -1 & +1 \end{pmatrix} \quad \text{(Gleichung 1)}$$

Hierbei bezeichnet "-1" einen Nichtmarkierungsbereich und "+1" einen Markierungsbereich, bestimmt also ob der jeweilige Blutbolus nicht-markiert oder markiert wird. Die Matrix M weist zu jeder Folge von markierten und/oder nicht-markierten Blutboli, das heißt zu jeder Zeile, eine dazu komplementäre Folge auf, wobei komplementäre Folgen unmittelbar hintereinander angeordnet sind. Zudem sind die einzelnen Folgen gemäß der Anzahl von Wechseln zwischen markierten- und nicht-markierten Blutboli sortiert. Das heißt, die Zeilen der Matrix M sind Walsh-sortiert. Die Walsh-Sortierung ist beispielsweise in Wolfram, S., 2002. *A new kind of science* 1st ed., Wolfram Media, Inc.und Wang, R., 2013. Sequency Ordered Walsh-Hadamard Matrix, http://fourier.eng.hmc.edu/e 16 1/lectures/wht/node3.html (Wolfram 2002) beschrieben.

Die Matrix M ist durch eine Kombination einer ersten Bestimmungsmatrix, die erfindungsgemäß eine Hadamard-Matrix ist, mit einer zweiten Bestimmungsmatrix, die erfindungsgemäß eine komplementäre Hadamard-Matrix, das heißt eine den komplementären Folgen von markierten und/oder nicht-markierten Fluidboli entsprechende, ist, gebildet, wobei zum Erzeugen der Matrix M wechselweise die Zeilen der Hadamard-Matrix und der komplementären Hadamard-Matrix verwendet werden. Die einzelnen Folgen von markierten und nicht-markierten Blutboli, die den einzelnen Zeilen der Matrix M entsprechen, sind schematisch und beispielhaft in der Fig. 2 illustriert. Fig. 2 zeigt 16 Folgen 10 von markierten Boli L und nicht-markierten Boli C, die auch als Kontrollboli aufgefasst werden könnten. In Fig. 2 entsprechen die mit ungeraden Zahlen (Zeilen 1, 3, 5,..., 15) indizierten Folgen, die auch als Folgen einer ersten Gruppe bezeichnet werden können, Zeilen der Hadamard-Matrix und die mit geraden Zahlen (Zeilen 2, 4, 6,..., 16) indizierten Folgen, die auch als Folgen einer zweiten Gruppe bezeichnet werden können, Zeilen der komplementären Hadamard-Matrix.

Die aus den ersten MR-Daten gewonnenen zweiten MR-Daten, die jeweils für einen zu einem bestimmten ersten Zeitpunkt erzeugten und zum zweiten Bereich geströmten Blutbolus indikativ sind, werden bevorzugt Voxel für Voxel bestimmt. Das heißt, die ersten MR-Daten sind bevorzugt erste MR-Bilder mit verschiedenen Voxeln, wobei des Weiteren ein erster Vektor und ein zweiter Vektor gebildet werden. Ein Vektorelement des ersten Vektors wird durch einen Voxelwert eines ersten MR-Bildes gebildet, wobei verschiedene Vektorelemente des ersten Vektors verschiedenen MR-Messungen entsprechen, die wiederum verschiedenen Folgen der ersten Gruppe (Zeilen 1, 3, 5,...,15) entsprechen. Ein Vektorelement des zweiten Vektors wird durch einen Voxelwert eines zweiten MR-Bildes gebildet, wobei verschiedene Vektorelemente des zweiten Vektors verschiedenen MR-Messungen entsprechen, die wiederum verschiedenen Folgen der zweiten Gruppe (Zeilen 2, 4, 6,...,16) entsprechen. Beispielsweise weist ein erster Vektor für den Voxel (1,1,1) die Voxelwerte für diesen Voxel in den unterschiedlichen ersten MR-Bildern auf, die in den unterschiedlichen MR-Messungen erzeugt worden sind, die den verschiedenen Folgen von Blutboli der ersten Gruppe (Zeilen 1, 3, 5,...,15) entsprechen. Für jeden Voxel werden daher bevorzugt zweite MR-Daten, i.e. Voxelwerte für den entsprechenden Voxel, bestimmt, die jeweils für einen zu einem bestimmten ersten Zeitpunkt erzeugten und zum zweiten Bereich geströmten Blutbolus indikativ sind, indem für jeden Voxel die inverse erste Bestimmungsmatrix mit dem ersten Vektor und die inverse zweite Bestimmungsmatrix mit dem zweiten Vektor multipliziert wird. Die beiden resultierenden Vektoren können beispielsweise gemittelt werden, wobei der resultierende gemittelte Vektor die zweiten MR-Daten beispielsweise für den Voxel (1,1,1) darstellt.

Des Weiteren ist die Auswerteeinheit 7 derart angepasst, dass zum Bestimmen der zweiten MR-Daten, die jeweils für einen - aus mehreren zu verschiedenen ersten Zeitpunkten erzeugten und zum zweiten Bereich geströmten Blutboli zusammengesetzten - Kombinations-Blutbolus und einen zeitlichen Abstand zwischen einem der ersten Zeitpunkte und dem zweiten Zeitpunkt indikativ sind, weniger erste MR-Daten kombiniert werden als für die Bestimmung der zweiten MR-Daten, die jeweils für einen zu einem bestimmten ersten Zeitpunkt erzeugten und zum zweiten Bereich geströmten einzelnen Blutbolus, indikativ sind. Dies wird im Folgenden unter Bezugnahme auf Fig. 2 beispielhaft erläutert.

In diesem Ausführungsbeispiel subtrahiert die Auswerteeinheit 7 das erste MR-Bild der ersten MR-Messung, das auf der ersten Zeile der ersten Gruppe (Zeile 1) der Blutbolifolgen 10 basiert, von dem ersten MR-Bild der zweiten MR-Messung, das auf der ersten Zeile der zweiten Gruppe (Zeile 2) der Blutbolifolgen 10 basiert, um das zweite MR-Bild 30, das heißt entsprechende zweite MR-Daten, zu erzeugen. Das zweite MR-Bild 30 ist indikativ für den Kombinations-Blutbolus, der aus allen acht einzelnen Blutboli gebildet wird, also die achtfache Länge eines einzelnen Blutbolus hat, und für den zeitlichen Abstand TI zwischen dem zweiten Zeitpunkt, zu dem die ersten MR-Bilder aufgenommen worden sind, und dem ersten Zeitpunkt, zu dem der Blutbolus 48 markiert wurde. Aus Sicht eines Kombinatons-Blutbolus könnten die einzelnen Blutboli auch als Unterboli oder Subboli bezeichnet werden. Die zweiten MR-Bilder 31, 32, 33 entsprechen jeweils einem Kombinations-Blutbolus, der aus vier einzelnen Blutboli gebildet wird, also die vierfache Länge eines einzelnen Blutbolus hat. Das zweite MR-Bild 31 ist erzeugt worden, indem das erste MR-Bild der vierten MR-Messung (4. Zeile) von dem ersten MR-Bild der ersten MR-Messung (1. Zeile) abgezogen worden ist. Das zweite MR-Bild 32 ist erzeugt worden, indem das erste MR-Bild der fünften MR-Messung (5. Zeile) von dem ersten MR-Bild der ersten MR-Messung (1. Zeile) abgezogen worden ist, und das zweite MR-Bild 33 ist erzeugt worden, indem das erste MR-Bild der dritten MR-Messung (3. Zeile) von dem ersten MR-Bild der ersten MR-Messung (1. Zeile) abgezogen worden ist. Das zweite MR-Bild 31 entspricht einem zeitlichen Abstand TI zwischen dem zweiten Zeitpunkt und dem Zeitpunkt zu dem der Blutbolus 49 markiert wurde. Das zweite MR-Bild 32 entspricht einem zeitlichen Abstand TI zwischen dem zweiten Zeitpunkt und dem Zeitpunkt zu dem der Blutbolus 50 markiert wurde und das zweite MR-Bild 33 entspricht einem zeitlichen Abstand TI zwischen dem zweiten Zeitpunkt und dem Zeitpunkt zu dem der Blutbolus 48 markiert wurde. Die Länge der einzelnen oder Kombinations-Blutboli, die zur Erzeugung der in Fig. 2 wiedergegebenen zweiten MR-Bilder 30-48 verwendet wurden, ist nochmals unter den entsprechenden Bildern in Form horizontaler Balken veranschaulicht. Das Subtrahieren und Addieren von nacheinander gemessenen MR-Bildern ist eine Grundprinzip bei zeitkodiertem ASL (vgl. Guenther 2012). Die hier vorgenommene, erfindungsgemäße Walsh-Sortierung der Bestimmungsmatrix sorgt automatisch dafür, dass mit zunehmender Anzahl von MR-Messungen immer kürzer werdende Kombinations-Blutboli den dekodierten zweiten MR-Bildern zu Grunde liegen.

Eine Methode zur Bestimmung der Dekodierungsvorschriften der zweiten MR-Bilder aus den ersten MR-Bildern für Matrizen beliebiger Größe und nach der beispielsweise auch die Bilder 30-48 bestimmt wurden, kann zum Beispiel wie folgt aussehen. Zunächst wird für jedes zweite MR-Bild, das jeweils für einen zu einem bestimmten ersten Zeitpunkt erzeugten und zum zweiten Bereich geströmten einzelnen Fluidbolus, das heißt Einzelfluidbolus, indikativ sind, symbolisch, das heißt in Symbolen geschrieben, aufgeschrieben, wie diese zweiten MR-Bilder $b_1,...,b_N$ sich aus ersten MR-Bildern $v_1,... v_N$ zusammensetzen, bzw. die $v_i$ zu den $b_i$ zusammengefügt werden.

Zum Beispiel anhand der ersten Gruppe (Zeilen 1, 3, 5,...,15) von Matrix M (siehe Gleichung 1) (analog kann auch für die zweite Gruppe (Zeilen 2, 4, 6,...,16 der Matrix M) vorgegangen werden):

$$b_2 = \frac{1}{8}(v_1 + v_2 + v_3 + v_4 - v_5 - v_6 - v_7 - v_8)$$

Wichtig ist dabei anzumerken, dass dieser Schritt symbolisch bereits vor der Erzeugung erster MR-Daten geschehen kann und dabei auch keine zweiten MR-Bilder erzeugt werden. Die symbolische Darstellung der einzelnen Fluidboli dient lediglich dazu arithmetische Rechenvorschriften herzuleiten für die zweiten MR-Bilder, die jeweils für einen zu einem bestimmten ersten Zeitpunkt erzeugten und zum zweiten Bereich geströmten Kombinations-Fluidbolus indikativ sind.

In einem zweiten Schritt werden aus den $b_i$ symbolisch durch Addieren und/oder Subtrahieren sämtliche gewünschten zweiten MR-Daten $K_i$ bestimmt. Dabei wird so vorgegangen, dass die Fluidboli für die die $b_i$ indikativ sind die Kombinations-Fluidboli ergeben für die die $K_i$ indikativ sind. Aus dieser symbolischen Schreibweise ergibt sich dann, nach Streichung aller sich zu Null wegehebenden Summanden, die arithmetische Rechenvorschrift für den gewünschten Kombinations-Fluidbolus.

Zum Beispiel Anhand der ersten Gruppe (Zeilen 1, 3, 5,..., 15) von Matrix M (siehe Gleichung 1 ) (analog kann auch für die zweite Gruppe (Zeilen 2, 4, 6,..., 16 von M) vorgegangen werden):

$$b_2 + b_3 = \frac{1}{8}((v_1 + v_2 + v_3 + v_4 - v_5 - v_6 - v_7 - v_8) + (v_1 + v_2 - v_3 - v_4 - v_5 - v_6 + v_7 + v_8))$$

$$= \frac{1}{4}(v_1 + v_2 - v_5 - v_6)$$

oder auch

$$b_5 + b_6 + b_7 + b_8 = \frac{1}{2}(v_1 - v_2)$$

Sollen zum Beispiel auch zweite MR-Bilder bestimmt werden, die für den ersten Fluidbolus oder Kombinations-Fluidboli, die den ersten Fluidbolus enthalten, indikativ sind, so werden erste MR-Bilder aus der ersten und der zweiten Gruppe kombiniert. In diesem Beispiel ist im Folgenden mit dem ersten Fluidbolus derjenige Fluidbolus gemeint, der den größten zeitlichen Abstand TI zwischen seiner Erzeugung und dem zweiten Zeitpunkt hat und mit dem letzten Fluidbolus derjenige Fluidbolus, der den kleinsten zeitlichen Abstand TI zwischen seiner Erzeugung und dem zweiten Zeitpunkt hat. Zur Bestimmung der zweiten MR-Bilder, die für den ersten Fluidbolus indikativ sind, werden einfach in der Rechenvorschrift für die zweiten MR-Bilder, die für den letzten Fluidbolus indikativ sind, die Summanden mit negativem Vorzeichen durch Einträge des zweiten Vektors, also Bildwerten der zur komplementären Matrix gehörenden ersten MR-Bilder $v_i^k$ (zweite Gruppe), mit den gleichen Indizes ersetzt. Analog können die Rechenvorschriften zur Bestimmung zweiter MR-Daten, die für Kombinations-Fluidboli, die den ersten Fluidbolus enthalten, indikativ sind, angepasst werden.

Zum Beispiel Anhand der ersten (Zeilen 1, 3, 5,..., 15) und zweiten (Zeilen 2, 4, 6,..., 16) Gruppe von Matrix M (siehe Gleichung 1):

$$b_1 + b_2 + b_3 + b_4 = \frac{1}{2}(v_1 - v_2^k)$$

Die Bestimmung von zweiten MR-Daten, die für den ersten Fluidbolus oder Kombinations-Fluidboli, die den ersten Fluidbolus enthalten, indikativ sind, ist also in diesem Beispiel erst durch die Kombination der Informationen der Bestimmungsmatrix und der hierzu komplementären Bestimmungsmatrix möglich geworden.

Die zweiten MR-Bilder, das heißt die zweiten MR-Daten, die Kombinations-Fluidboli entsprechen, oder genauer für diese indikativ sind, werden mit weniger ersten MR-Daten erzeugt als die zweiten MR-Daten, die jeweils einzelnen Fluidboli entsprechen. Insbesondere werden in letzterem Fall für die jeweils acht einzelnen Fluidboli, das heißt für die dazugehörigen acht ersten Zeitpunkte, zweite MR-Daten bestimmt, indem zumindest acht erste MR-Bilder, insbesondere im Falle der oben genannten zusammengesetzten Bestimmungsmatrix (M, vgl. Gleichung 1) sämtliche 16 erste MR-Bilder, die den 16 Folgen 10 entsprechen, durch Verwendung der oben genannten ersten und zweiten Bestimmungsmatrizen zusammengefügt werden. Für die Bestimmung der zweiten MR-Daten, die den Kombinations-Fluidboli entsprechen, werden dagegen beispielsweise nur zwei erste MR-Bilder benötigt, beispielsweise die auf den Zeilen 1 und 2 der Bestimmungsmatrix M basierenden ersten MR-Bilder. Die zweiten MR-Daten, die Kombinations-Fluidboli entsprechen, können daher erzeugt werden, selbst wenn nicht sämtliche 16 ersten MR-Bilder aufgenommen worden sind. Das heißt, die zweiten MR-Bilder, die den Kombinations-Fluidboli entsprechen, können bereits erzeugt werden, bevor sämtliche 16 ersten MR-Bilder gemessen worden sind. Zum Beispiel können bereits während der Messung der 16 ersten MR-Bilder zweite MR-Bilder, insbesondere in Echtzeit, erzeugt werden. Des Weiteren können die zweiten MR-Bilder selbst dann erzeugt werden, wenn beispielsweise aufgrund von Bildartefakten oder anderen Störungen nicht sämtliche 16 erste MR-Bilder gemessen werden konnten.

Mit den zweiten MR-Bildern kann dann beispielsweise ein MR-Signal zu verschiedenen Zeitpunkten bestimmt und somit voxel-/pixelweise der Signalverlauf über die Zeit ermittelt werden. Aus diesen Signal-Zeit-Kurven lassen sich dann voxel-/pixelweise verschiedene, für die klinische Diagnose relevante Parameter bestimmen. Beispiele dafür werden zum Beispiel in Buxton, R.B. et al., 1998. A general kinetic model for quantitative perfusion imaging with arterial spin labeling. Magnetic Resonance in Medicine, 40(3), pp.383-96 beschrieben.

Das in Fig. 1 gezeigte MR-System 1 weist des Weiteren eine Steuerungseinheit 8 auf, die angepasst ist, die verschiedenen Einheiten des MR-Systems 1 zu steuern, und eine Eingabeeinheit 9 wie beispielsweise eine Tastatur, eine Computermaus, ein Tastbildschirm et cetera. Zudem umfasst das MR-System 1 eine Ausgabeeinheit 25 wie beispielsweise einen Monitor.

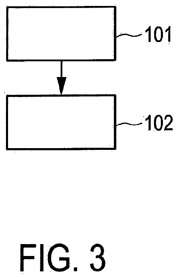

Im Folgenden wird ein MR-Verfahren zum Erzeugen von MR-Daten eines strömenden Fluids unter Bezugnahme auf ein Flussdiagramm beschrieben, das in Fig. 3 gezeigt ist.

In Schritt 101 werden erste MR-Daten erfasst, wobei mehrere MR-Messungen durchgeführt werden und wobei in jeder MR-Messung erste MR-Daten gemessen werden. Insbesondere wird in jeder MR-Messung ein erstes MR-Bild erzeugt, um erste MR-Daten zu messen. Bei der Erzeugung der ersten MR-Daten wird zu verschiedenen ersten Zeitpunkten in einem ersten Bereich das Fluid entweder magnetisch markiert oder nicht markiert, um eine strömende Folge von markierten- und/oder nicht-markierten Fluidboli zu erzeugen. Zudem werden zu einem zweiten Zeitpunkt in einem zweiten Bereich, zu dem das Fluid ausgehend von dem ersten Bereich strömt, die ersten MR-Daten, die indikativ sind für die erzeugte Folge von markierten und/oder nicht-markierten Fluidboli, gemessen. Das heißt, in jeder MR-Messung wird eine jeweilige Folge von markierten und/oder nicht-markierten Fluidboli geschaffen und für jede dieser Folgen wird bevorzugt ein erstes MR-Bild als Messung der ersten MR-Daten erzeugt.

Zudem werden in Schritt 101 zweite MR-Daten bestimmt, die jeweils für einen - aus mehreren zu verschiedenen ersten Zeitpunkten erzeugten und zum zweiten Bereich geströmten markierten/nicht-markierten Fluidboli zusammengesetzten - Kombinations-Fluidbolus und einen zeitlichen Abstand TI zwischen einem der ersten Zeitpunkte und dem zweiten Zeitpunkt indikativ sind, indem die in den verschiedenen MR-Messungen gemessenen ersten MR-Daten, wie bereits beschrieben, zusammengefügt werden. Da für die Bestimmung dieser zweiten MR-Daten nicht sämtliche ersten MR-Daten notwendig sind, können schon während der Akquisition der ersten MR-Daten, das heißt in diesem Ausführungsbeispiel der ersten MR-Bilder, zweite MR-Daten für die Kombinations-Fluidboli bestimmt werden.

Nachdem die Messung der ersten MR-Daten abgeschlossen ist, können diese in Schritt 102 zudem auch dazu verwendet werden, diejenigen zweite MR-Daten zu bestimmen, die jeweils für einen zu einem bestimmten ersten Zeitpunkt erzeugten und zum zweiten Bereich geströmten einzelnen Fluidbolus indikativ sind.

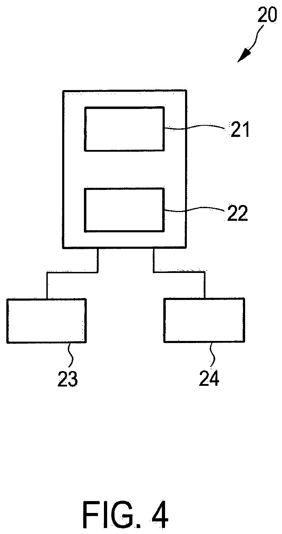

Fig. 4 zeigt schematisch und beispielhaft ein Auswertesystem 20 zum Auswerten von MR-Daten eines strömenden Fluids. Das Auswertesystem 20 umfasst eine Datenbereitstellungseinheit 21 zum Bereitstellen von ersten MR-Daten, wobei die Datenbereitstellungseinheit 21 angepasst ist, erste MR-Daten mehrerer MR-Messungen bereitzustellen und wobei in jeder MR-Messung erste MR-Daten gemessen wurden, in dem a) zu verschiedenen ersten Zeitpunkten in einem ersten Bereich das Fluid entweder magnetisch markiert oder nicht markiert wurde, um eine strömende Folge von markierten und/oder nicht-markierten Fluidboli zu erzeugen, und b) zu einem zweiten Zeitpunkt in einem zweiten Bereich, zu dem das Fluid ausgehend von einem ersten Bereich strömt, die ersten MR-Daten, die indikativ sind für die erzeugte Folge von markierten und/oder nicht-markierten Fluidboli, gemessen wurden. Das Auswertesystem 20 umfasst des Weiteren eine Auswerteeinheit 22, die der oben beschriebenen Auswerteeinheit 7 des MR-Systems 1 entspricht, zum Auswerten der in den verschiedenen MR-Messungen gemessenen ersten MR-Daten. Die Auswerteeinheit 22 ist angepasst, zweite MR-Daten zu bestimmen, die jeweils für einen - aus mehreren zu verschiedenen ersten Zeitpunkten erzeugten und zum zweiten Bereich geströmten markierten/nicht-markierten Fluidboli zusammengesetzten - Kombinations-Fluidbolus und einen zeitlichen Abstand zwischen einem der ersten Zeitpunkte und dem zweiten Zeitpunkt indikativ sind, basierend auf der bereits beschriebenen Zusammenfügung der in den verschiedenen MR-Messungen gemessenen ersten MR-Daten. Die Auswerteeinheit 22 ist bevorzugt auch angepasst, zweite MR-Daten zu bestimmen, die jeweils für einen zu einem bestimmten ersten Zeitpunkt erzeugten und zum zweiten Bereich geströmten einzelnen Fluidbolus indikativ sind, basierend auf einer Kombination der in den verschiedenen MR-Messungen gemessenen ersten MR-Daten. Zudem umfasst das Auswertesystem 20 eine Eingabeeinheit 23, wie beispielsweise eine Tastatur, eine Computermaus oder einen Tastbildschirm, und eine Ausgabeeinheit 24 wie beispielsweise einen Monitor.

Obwohl in den oben beschriebenen Ausführungsformen die ersten MR-Daten nur an einem zweiten Zeitpunkt gemessen worden sind, können die ersten MR-Daten in anderen Ausführungsformen auch an mehreren unterschiedlichen zweiten Zeitpunkten gemessen werden.

Beispielsweise können erste MR-Daten in jeder MR-Messung auch an mehreren zweiten Zeitpunkten $t_1<t_2<t_3<...$ gemessen werden. Für jeden Zeitpunkt $t_i$ können dann zweite MR-Daten erzeugt werden. Damit könnte dann zum Beispiel die Signalintensität an mehr Zeitpunkten ermittelt werden, nämlich dem Produkt der Anzahl von zweiten Zeitpunkten und der Anzahl von zweiten MR-Daten, als es mit einer Messung zu nur einem zweiten Zeitpunkt möglich wäre. Damit stünden mehr Datenpunkte für eine Signal-Zeit-Kurve zur Verfügung, was zum Beispiel ein Fitting verbessern kann.

Es folgen noch einige beispielhafte Erläuterungen zu einzelnen Aspekten der Offenbarung.

Beispielsweise kann die Datenerfassungseinheit angepasst sein, zur Markierung des Blutes zu einem ersten Zeitpunkt und in einem gewählten Abschnitt, z.B. der Halsschlagader, eine Drehung der Blutmagnetisierung um einen gewünschten Winkel zu bewirken. In einer beispielhaften Ausführung wird dazu die aus den Spins der Protonen im Blut resultierende Magnetisierung aus dem relaxierten Zustand (0°) um 180° gedreht (invertiert). Das wird hier auch Markierung oder labeling genannt. Auf diese Art und Weise wird ein klar definierter Längenabschnitt des fließenden Blutes (Bolus, Markierungsbereich) erzeugt. Das Blut mit invertierter Magnetisierung verändert dort, wo es hinfließt, lokal die magnetischen Eigenschaften und somit den Kontrast eines nach dem labeling erzeugten MR Bildes (label-Bild). Da diese Veränderungen sehr klein und mit bloßem Auge nicht erkennbar sind, wird zusätzlich ein Bild ohne vorheriges labeling erzeugt, das sog. control-Bild. In der Differenz von label- und control-Bild werden zum Beispiel die Effekte von Perfusion und/oder Fluss dann sichtbar und gegebenenfalls auch quantifizierbar. In einer weiteren beispielhaften Ausführung wird statt einer Inversion eine Sättigung (Drehung um 90°) der Magnetisierung bewirkt.

Die Drehung (auch Neigung oder Flip genannt) der Magnetisierung kann zum Beispiel durch Radiofrequenzpulse (RF-Pulse) erfolgen. Ein Beispiel für solche RF-Pulse wird in Payne, G.S. & Leach, M.O., 1997. Implementation and evaluation of frequency offset corrected inversion (FOCI) pulses on a clinical MR system. Magnetic resonance in medicine, 38(5), pp.828-33 und Dai, W. et al., 2008. Continuous flow-driven inversion for arterial spin labeling using pulsed radio frequency and gradient fields. Magnetic resonance in medicine, 60(6), pp.1488-97 beschrieben.

Diese Technik zur Markierung des Blutes kann zum Beispiel dafür verwendet werden vor der Messung eines MR-Bildes nicht nur einen einzelnen markierten oder nicht-markierten Fluidbolus zu erzeugen. Es können vielmehr Folgen von markierten und nicht-markierten Fluidboli geschaffen werden und es können mehrere MR-Messungen erster MR-Daten nacheinander ausgeführt werden, wobei potentiell in jeder Messung eine unterschiedliche Folge von markierten und nicht-markierten Fluidboli erzeugt wird. Die konkrete Gestalt dieser Folgen kann zum Beispiel in Form einer Matrix vorgegeben werden, wobei die Matrixeinträge die Art der Markierung (zum Beispiel markiert oder nicht markiert) vorgeben und jede Zeile der Matrix einer MR-Messung erster MR-Daten entspricht.

Für die beispielhafte Beschreibung der Erzeugung von ersten MR-Daten wird auch auf Gadian, D.G., 1996. NMR & Its Applications to Living Systems 2 Sub., Oxf.U.P.; Bernstein, M., King, K. & Zhou, X., 2004. Handbook of MRI pulse sequences, Academic Press verwiesen. Für weitere Details des Markierungsprozesses und der Erzeugung der ersten MR-Daten wird beispielhaft auf Williams, D.S. et al., 1992. Magnetic resonance imaging of perfusion using spin inversion of arterial water. Proceedings of the National Academy of Sciences of the United States of America, 89(1), pp.212-6, Detre, J., Leigh, J. & Williams, D., 1996. Perfusion imaging. Magnetic Resonance in Medicine, 35(1), pp.70-9, Günther, M., 2007. Habilitationsschrift. Ruprechts-Karl-Universität Heidelberg (Günther 2007) und Günther, M., Oshio, K. & Feinberg, D.A., 2005. Single-shot 3D imaging techniques improve arterial spin labeling perfusion measurements. Magnetic resonance in medicine, 54(2), pp.491-498 verwiesen.

Beispielsweise können zur Gewinnung zweiter MR-Daten, die für einen Kombinations-Blutbolus indikativ sind (im Folgenden auch zweite MR-Kombinationsdaten genannt), aus ersten MR-Daten nur Teilmengen aller ersten MR-Daten herangezogen werden und diese trotzdem für die klinische Diagnostik relevant sein. In anderen Worten sind in diesem Beispiel für die Rekonstruktion von zweiten MR-Kombinationsdaten weniger erste MR-Daten nötig als zur Rekonstruktion zweiter MR-Daten, denen einzelne Fluidboli zu Grunde liegen (im Folgenden zweite MR-Einzeldaten genannt). Dem liegt zum Beispiel folgende Beobachtung zu Grunde. Schreibt man die Berechnung der zweiten MR-Kombinationsdaten aus zweiten MR-Einzeldaten in Symbolen arithmetisch auf, erschließt sich diese Beobachtung. Werden nämlich bei dieser Berechnung die zweite MR-Einzeldaten, wiederum in Symbolen, als Kombination von Addition und Subtraktion der ersten MR-Daten geschrieben, ergibt sich, dass sich einige der ersten MR-Daten paarweise zu Null berechnen. Aus dieser Darstellung ergibt sich, dass die zweiten MR-Kombinationsdaten als Kombination von Addition und Subtraktion von Teilmengen von ersten MR-Daten beschrieben werden können und, dass für die Bestimmung zweiter MR-Kombinationsdaten weniger erste MR-Daten notwendig sind, als zur Bestimmung zweite MR-Einzeldaten. In Sinne dieses Beispiels, werden die zweiten MR-Kombinationsdaten direkt aus den ersten MR-Daten erzeugt (und nicht über den Umweg der Erzeugung von zweiten MR-Einzeldaten). Dies kann gegenüber dem Stand der Technik vorteilhaft sein, da beispielsweise die in der Auswerteeinheit vollzogene Berechnung von zweiten MR-Kombinationsdaten beschleunigt werden kann. Denn es muss nicht mehr wie in (Günther 2007) der Umweg über die Berechnung der zweite MR-Einzeldaten erfolgen. Die zweiten MR-Einzeldaten müssen also nur dann erzeugt werden, wenn die höchst mögliche Detailauflösung, hier insbesondere Zeitauflösung, verlangt wird. Weiter kann zum Beispiel so auch die Robustheit der Messung erhöht werden. Wenn beispielsweise einige erste MR-Daten fehlerhaft, zum Beispiel weil sich ein Patient bewegt hat, oder nicht verfügbar sein sollten, können gegebenenfalls. zweite MR-Einzeldaten nicht oder nur fehlerhaft bestimmt werden. Trotzdem könnten dann aus den intakten und verfügbaren ersten MR-Daten immer noch zweite MR-Kombinationsdaten bestimmt werden.

Ist beispielsweise die Bestimmungsmatrix invertierbar, das heißt die zu ihr inverse Matrix kann bestimmt werden, ist es möglich mit Hilfe dieser und dem Vektor bestehend aus ersten MR-Werten zweite MR-Einzel- und Kombinationsdaten zu bestimmen. Es sind aber auch andere beispielhafte Wege denkbar, um aus ersten MR-Daten zweite MR-Einzel- und MR-Kombinationsdaten zu bestimmen. Ein Beispiel ist die weiter oben erläuterte beispielhafte arithmetische Darstellung der zweiten MR-Kombinationsdaten als symbolisch geschriebene Kombination von Addition und/oder Subtraktion von ersten MR-Daten.

Ein Beispiel für eine denkbare Bestimmungsmatrix ist die Hadamard-Matrix, die unter anderem in (Guenther 2012) oder (Wolfram 2002) beschrieben wird.

Beispielsweise kann bei der Wahl der Bestimmungsmatrizen zu einer Ursprungsmatrix auch die zu ihr komplementäre Matrix (Komplementärmatrix), bei der markierte und nicht markierte Zustände vertauscht sind, hinzugezogen werden. Dies hat zum Beispiel folgenden Vorteil gegenüber dem Stand der Technik. Gewöhnlich können aus N ersten MR-Daten nur N-1 zweite MR-Daten bestimmt werden, beispielsweise können zweite MR-Daten, die für den ersten Fluidbolus indikativ sind nicht erzeugt werden. Verwendet man jedoch die Informationen aus der Ursprungsmatrix und der Komplementärmatrix können zweite MR-Einzeldaten, die für alle N Fluidboli indikativ sind, erzeugt werden. Weiter können dann auch zweite MR-Kombinationsdaten, die für Kombinations-Fluidboli, die den ersten Fluidbolus enthalten würden, indikativ sind, aus den ersten MR-Daten erzeugt werden. Dies kann zum Beispiel auch erlauben, aus zwei MR-Messungen, die der ersten Zeile der Ursprungsmatrix und die der ersten Zeile der Komplementärmatrix zweite MR-Kombinationsdaten, die für den Kombinations-Fluidbolus, der aus allen N einzelnen Fluidboli besteht, indikativ sind, zu erzeugen. Eine weitere denkbare Ausführung der Bestimmungsmatrix könnte die Gestalt haben, dass nur bestimmte Zeilen einer Ursprungsmatrix mit den Zeilen der zu ihr komplementären Matrix alternieren. Die restlichen Zeilen der Ursprungsmatrix stünden dann nicht alternierend und in der Reihenfolge in der sie in der Ursprungsmatrix stehen in der Bestimmungsmatrix. An anderer Stelle der Bestimmungsmatrix stünden zudem die restlichen Zeilen der Komplementärmatrix, nicht alternierend und in der Reihenfolge in der sie in der Komplementärmatrix stehen.

Ein Beispiel für die Ordnung nach der Anzahl von Wechseln zwischen markierten und nicht-markierten Fluidboli ist die sog. Sequenz-Sortierung. Dabei sind die Zeilen nach ihrer Sequenz geordnet und die Sequenz wird definiert als die Anzahl der Vorzeichenwechsel (i.e. Nulldurchgänge) der Einträge der Zeile. Im Falle von Sequenz-sortierten Hadamard-Matrizen kann in diesem Zusammenhang auch von einer Walsh-Sortierung gesprochen werden (siehe dazu zum Beispiel (Wolfram 2002)). Mit eine Sequenz-sortierten Bestimmungsmatrix kann beispielsweise die Abfolge der MR-Messungen so umsortiert werden, dass die zur Erzeugung von zweiten MR-Kombinationsdaten notwendigen ersten MR-Daten möglichst früh gemessen werden. Ein Vorteil davon gegenüber dem Stand der Technik ist zum Beispiel dass grundsätzlich, bereits bevor sämtliche erste MR-Daten gemessen worden sind, die zur Bestimmung der zweiten MR-Einzeldaten benötigt werden, zweite MR-Kombinationsdaten bestimmt werden könnten und diese bereits relevant für die klinische Diagnostik sind. Es ist beispielsweise denkbar, dass in Echtzeit bereits während der Messung die zweiten MR-Kombinationsdaten bestimmt werden, denn werden die ersten MR-Daten in geeigneter Reihenfolge erzeugt, könnten daraus zweite MR-Kombinationsdaten bereits während der Messung erzeugt werden. Das erlaubt es zum Beispiel dem die Messung begleitenden medizinischen Personal erste Informationen zu einem sehr frühen Zeitpunkt der Untersuchung zu erhalten. Gegebenenfalls kann dann die laufende Messung verändert werden und/oder weitere diagnostische Schritte geplant werden. Auch hier kann so zum Beispiel die Robustheit der Messung erhöht werden. Patientenbewegungen, die die Bilder unbrauchbar machen können, nehmen gewöhnlich mit der Messzeit zu. Werden daher zu einem möglichst frühen Zeitpunkt, die Teilmengen von ersten MR-Daten erzeugt, die bereits eine Erzeugung von zweiten MR-Kombinationsdaten erlauben, sinkt die Wahrscheinlichkeit, dass auf Grund von Patientenbewegung sämtliche zweiten MR-Einzel- und/oder MR-Kombinationsdaten nicht oder nur fehlerhaft erzeugt werden können.

In anderen Worten lässt sich also sagen, dass beispielsweise ermöglicht werden kann die Reihenfolge der MR-Messungen und/oder der Auswerteschritte so zu wählen, dass zunächst die zweiten MR-Daten bestimmt werden, die aus einer möglichst geringen Anzahl von ersten MR-Daten erzeugt werden können. Diese Anzahl kann sogar minimal sein.

Ein allgemeines Beispiel für eine Walsh-Sortierung wird im Folgenden gegeben.

Die Walsh-Sortierung kann in drei Schritten durchgeführt werden. Dazu wird eine gegebene Sequenz $S$ zunächst als Binärzahl (auch Dualzahl genannt) geschrieben:

$$S = (S_{n-1}S_{n-2}...S_1S_0)_2 = \sum_{i=0}^{n-1} S_i 2^i$$

Diese Zahl im Binärcode wird dann in Graycode konvertiert:

$$g_i = S_i \oplus S_{i+1}, i \in [0, n-1], S_n \equiv 0$$

Dabei steht $\oplus$ für das exklusive Oder.

Durch eine Bitumkehr

$$k_i = g_{n-1-i}$$

werden dann aus den $g_i$'s die binären Werte $k_i$ ermittelt. Die zur Sequenz gehörige Zeilennummer $k$ der Hadamardmatrix kann dann aus der binären Darstellung

$$k = (k_{n-1}k_{n-2}...k_1k_0)_2 = \sum_{i=0}^{n-1} k_i 2^i$$

ermittelt werden. Werden die Zeilen k der Hadamardmatrix so angeordnet, dass die zu den k gehörenden Sequenzen S auf- oder absteigen nennt man die Matrix Walsh-sortiert.

Insbesondere für N=8 Zeilen ergeben die Schritte:

| S | 0 | 1 | 2 | 3 | 4 | 5 | 6 | 7 |
|---|---|---|---|---|---|---|---|---|
| Binär | 000 | 001 | 010 | 011 | 100 | 101 | 110 | 111 |
| Gray code | 000 | 001 | 011 | 010 | 110 | 111 | 101 | 100 |
| Bit-reverse | 000 | 100 | 110 | 010 | 011 | 111 | 101 | 001 |
| k | 0 | 4 | 6 | 2 | 3 | 7 | 5 | 1 |

Wenn beispielsweise auch quantitative Perfusionsdaten bestimmt werden sollen (siehe zum Beispiel (Buxton et al. 1998)), kann die Auswerteeinheit auch weitere arithmetische Rechenoperationen zur Bildweiterverarbeitung ermöglichen.

Der Vollständigkeit halber wird noch auf die folgende Literatur hingewiesen.

Ein zu Guenther 2012 sehr ähnliches Verfahren wird in der US-Patentschrift US 8,610,433 B2 beschrieben.

In Guenther 2007 wird vorgeschlagen einzelne Boli (Markierungsbereiche), dort Subboli genannt, zu längeren, sog. virtuellen (Sub)Boli zusammenzufassen. Denn aus der Summe mehrerer (Sub)Boli kann ein beliebiger, längerer virtueller (Sub)Bolus erzeugt werden und auch der Abstand zur Bildauslese, die TI, dort auch post labeling delay (PLD) genannt, kann so zusätzlich variiert werden. In der Praxis werden dazu zunächst alle zu den einzelnen (Sub)Boli gehörenden rekonstruierten MR-Bilder aus den gemessenen MR-Bildern rekonstruiert und diese dann in einem weiteren Rekonstruktionsschritt zu zweifach rekonstruierten MR-Bildern addiert, so dass die zu den rekonstruierten MR-Bildern gehörenden (Sub)Boli zu den zweifach rekonstruierten MR-Bildern gehörende virtuelle (Sub)Boli bilden.

Dies und weitere Aspekte werden ausdrücklich in (Günther 2007), Dai, W., Shankaranarayanan, A. & Alsop, D.C., 2013. Volumetric measurement of perfusion and arterial transit delay using hadamard encoded continuous arterial spin labeling. Magnetic resonance in medicine, 69(4), pp.1014-22 und Teeuwisse, W.M. et al., 2014. Time-encoded pseudocontinuous arterial spin labeling: Basic properties and timing strategies for human applications. Magnetic resonance in medicine beschrieben.

In den Ansprüchen schließen die Wörter "aufweisen" und "umfassen" nicht andere Elemente oder Schritte aus und der unbestimmte Artikel "ein" schließt eine Mehrzahl nicht aus.

Eine einzelne Einheit oder Vorrichtung kann die Funktionen mehrerer Elemente durchführen, die in den Ansprüchen aufgeführt sind. Die Tatsache, dass einzelne Funktionen und Elemente in unterschiedlichen abhängigen Ansprüchen aufgeführt sind, bedeutet nicht, dass nicht auch eine Kombination dieser Funktionen oder Elemente vorteilhaft verwendet werden könnte.

Die Steuerung des MR-System und/oder des Auswertesystems gemäß des MR-Verfahrens bzw. des Auswerteverfahrens kann als Programmcode eines Computerprogramms und/oder als entsprechende Hardware implementiert sein.

Ein Computerprogramm kann auf einem geeigneten Medium gespeichert und/oder verteilt werden, wie beispielsweise einem optischen Speichermedium oder einem Festkörperspeichermedium, das zusammen mit oder als Teil anderer Hardware vertrieben wird. Das Computerprogramm kann aber auch in anderen Formen vertrieben werden, beispielsweise über das Internet oder andere Telekommunikationssysteme.

Die Bezugszeichen in den Ansprüchen sind nicht derart zu verstehen, dass der Gegenstand und der Schutzbereich der Ansprüche durch diese Bezugszeichen eingeschränkt sind.


**Patentansprüche**

1. System (1, 20) zum Auswerten von diagnostisch verwendbaren Magnetresonanz-daten ($K_i$, $b_i$) eines strömenden Fluids, wobei das System (1, 20) aufweist:

   - eine Datenerfassungseinheit (5), die angepasst ist, mehrere Magnetresonanzmessungen durchzuführen und dabei in jeder Magnetresonanzmessung erste Magnetresonanzdaten ($v_i$) misst, indem in jeder Magnetresonanzmessung

      a) zu unterschiedlichen ersten Zeitpunkten in einem ersten Bereich das Fluid entweder magnetisch markiert oder nicht markiert wird, derart, dass eine strömende Folge (10) von markierten (L) und/oder nicht-markierten (C) Fluidboli entsteht, und

      b) zu einem zweiten Zeitpunkt in einem zweiten Bereich, zu dem das Fluid ausgehend von dem ersten Bereich strömt, die ersten Magnetresonanzdaten ($v_i$), die indikativ sind für die erzeugte Folge (10) von markierten und/oder nicht-markierten Fluidboli (L, C), gemessen werden, wobei die Datenerfassungseinheit (5) angepasst ist, zu jeder Folge (10) von markierten und/oder nicht-markierten Fluidboli (L, C) eine komplementäre Folge von markierten und/oder nicht-markierten Fluidboli (L, C) zu erzeugen, wobei die Magnetresonanzmessungen der ersten Magnetresonanzdaten ($v_i$) von zueinander komplementären Folgen (10) unmittelbar nacheinander durchgeführt werden, und

      die so gemessenen ersten Magnetresonanzdaten ($v_i$) bereitzustellen, oder
      - eine Datenbereitstellungseinheit (21), die angepasst ist, gemäß a) und b) gemessene erste Magnetresonanzdaten ($v_i$) bereitzustellen, und
      - eine Matrixbereitstellungseinheit (6) zum Bereitstellen einer zweiwertigen Bestimmungsmatrix, wobei eine Zeile der Bestimmungsmatrix eine Folge (10) von markierten und/oder nicht-markierten Fluidboli (L, C) repräsentiert und unterschiedliche Zeilen der Bestimmungsmatrix unterschiedlichen Magnetresonanzmessungen der ersten Magnetresonanz-Daten ($v_i$) zugrunde gelegt werden, wobei die Bestimmungsmatrix durch eine Kombination einer Walsh-sortierten Hadamard-Matrix mit einer komplementären Walsh-sortierten Hadamard-Matrix gebildet ist, wobei die gebildete Matrix wechselweise die Zeilen der Walsh-sortierten Hadamard-Matrix und der komplementären Walsh-sortierten Hadamard-Matrix aufweist,
      - eine Auswerteeinheit (7, 22), die angepasst ist, basierend auf einer Zusammenfügung der in den verschiedenen Magnetresonanzmessungen gemessenen ersten Magnetresonanzdaten ($v_i$) unter Berücksichtigung der Bestimmungsmatrix,

      i) zweite Einzelfluidbolus-Magnetresonanzdaten ($b_i$; 41-48) zu bestimmen, die jeweils für einen zu einem bestimmten ersten Zeitpunkt erzeugten und zum zweiten Bereich geströmten einzelnen Fluidbolus (L, C) indikativ sind, basierend auf einer Zusammenfügung der in den verschiedenen Magnetresonanzmessungen gemessenen ersten Magnetresonanzdaten ($v_i$), und

      ii) zweite Kombinationsfluidbolus-Magnetresonanzdaten ($K_i$; 30-40) zu bestimmen, die jeweils für einen Kombinations-Fluidbolus und einen zeitlichen Abstand (TI) zwischen einem der ersten Zeitpunkte und dem zweiten Zeitpunkt indikativ sind, wobei der Kombinations-Fluidbolus aus mehreren zu verschiedenen ersten Zeitpunkten markierten/nicht-markierten und zum zweiten Bereich geströmten Fluidboli (L, C) zusammengesetzt ist, wobei

- die Datenerfassungseinheit (5) angepasst ist, die ersten Magnetresonanzdaten ($v_i$) in einer Reihenfolge zu messen, die es ermöglicht, dass, bereits bevor sämtliche erste Magnetresonanzdaten ($v_i$) gemessen worden sind, die zur Bestimmung der zweiten Einzelfluidbolus-Magnetresonanzdaten ($b_i$; 41-48) benötigt werden, in der Auswerteeinheit (7, 22) zweite Kombinationsfluidbolus-Magnetresonanzdaten ($K_i$; 30-40) bestimmt werden können, **dadurch gekennzeichnet, dass**

die Walsh-sortierte Hadamard-Matrix und die komplementäre Walsh-sortierte Hadamard-Matrix jeweils einen Grad $2^k$ mit $k \geq 2$ aufweisen, und

- die Auswerteeinheit (7, 22) angepasst ist, die ersten Magnetresonanzdaten ($v_i$) in einer Reihenfolge auszuwerten, die es ermöglicht, dass, bereits bevor sämtliche erste Magnetresonanzdaten ($v_i$) gemessen worden sind, die zur Bestimmung der zweiten Einzelfluidbolus-Magnetresonanzdaten ($b_i$; 41-48) benötigt werden, zweite Kombinations-fluidbolus-Magnetresonanzdaten ($K_i$; 30-40) bestimmt werden können, wobei für die Bestimmung von zweiten Magnetresonanzdaten für einen Kombinationsfluidbolus weniger erste Magnetresonanzdaten verwendet werden als für die Bestimmung von zweiten Magnetresonanzdaten für einen Einzelfluidbolus.

2. System (1, 20) nach Anspruch 1, **dadurch gekennzeichnet, dass** die Auswerteeinheit (7, 22) angepasst ist, die ersten Magnetresonanzdaten ($v_i$) in einer Reihenfolge auszuwerten, bei der die Anzahl von Wechseln zwischen markierten und nicht-markierten Fluidboli (L, C) in den zu den jeweiligen, in die Auswertung einfließenden ersten Magnetresonanzdaten ($v_i$) gehörenden Fluidboli-Folgen (10) zunimmt.

3. System (1, 20) nach Anspruch 1 oder 2, **dadurch gekennzeichnet, dass** die Zeilen der Walsh-sortierten Hadamard-Matrix eine erste Gruppe von Folgen (10) von markierten/nicht-markierten Fluidboli (L, C) repräsentieren, wobei unterschiedliche Zeilen der Walsh-sortierten Hadamard-Matrix unterschiedlichen Magnetresonanzmessungen der ersten Magnetresonanzdaten ($v_i$) zugrunde gelegt werden, und die Zeilen der komplementären Walsh-sortierten Hadamard-Matrix eine zweite Gruppe von Folgen (10) von markierten/nicht-markierten Fluidboli (C, L) repräsentieren, die komplementär sind zu den Fluidboli-Folgen der ersten Gruppe, wobei unterschiedliche Zeilen der komplementären Walsh-sortierten Hadamard-Matrix unterschiedlichen Magnetresonanzmessungen der ersten Magnetresonanzdaten ($v_i$) zugrunde gelegt werden, wobei die Auswerteeinheit (7, 22) des Weiteren angepasst ist,

- einen ersten Vektor zu bilden, dessen Vektorelemente jeweils durch einen Magnetresonanzwert der ersten Magnetresonanzdaten ($v_i$) gebildet werden und verschiedene Vektorelemente des ersten Vektors verschiedenen Magnetresonanzmessungen der ersten Magnetresonanzdaten ($v_i$) entsprechen, die wiederum verschiedenen Fluidboli-Folgen der ersten Gruppe entsprechen,
- einen zweiten Vektor zu bilden, wobei ein Vektorelement des zweiten Vektors durch einen Magnetresonanzwert der ersten Magnetresonanzdaten ($v_i$) gebildet wird und verschiedene Vektorelemente des zweiten Vektors verschiedenen Magnetresonanzmessungen der ersten Magnetresonanzdaten ($v_i$) entsprechen, die wiederum verschiedenen Fluidboli-Folgen der zweiten Gruppe entsprechen,
- die Walsh-sortierte Hadamard-Matrix und die komplementäre Walsh-sortierte Hadamard-Matrix zu invertieren, und
- die zweiten Magnetresonanzdaten zu bestimmen, indem die invertierte Walsh-sortierte Hadamard-Matrix auf den ersten Vektor und die invertierte komplementäre Walsh-sortierte Hadamard-Matrix auf den zweiten Vektor angewendet wird.

4. Verfahren zum Auswerten von diagnostisch verwendbaren Magnetresonanzdaten eines strömenden Fluids, wobei das Verfahren aufweist:

- Erfassen und Bereitstellen von ersten Magnetresonanzdaten ($v_i$), wobei mehrere Magnetresonanzmessungen durchgeführt werden und dabei in jeder Magnetresonanzmessung erste Magnetresonanzdaten ($v_i$) gemessen werden, indem in jeder Magnetresonanzmessung

a) zu unterschiedlichen ersten Zeitpunkten in einem ersten Bereich des Fluides dieses entweder magnetisch markiert oder nicht markiert wird, derart, dass eine strömende Folge (10) von markierten (L) und/oder nicht-markierten (C) Fluidboli erzeugt wird, und
b) zu einem zweiten Zeitpunkt an einem zweiten Ort, zu dem das Fluid ausgehend von dem ersten Bereich strömt, die ersten Magnetresonanzdaten ($v_i$), die indikativ sind für die erzeugte Folge (10) von markierten und/oder nicht-markierten Fluidboli (L, C), gemessen werden, wobei zu jeder Folge (10) von markierten und/oder nicht-markierten Fluidboli (L, C) eine komplementäre Folge von markierten und/oder nicht-markierten Fluidboli (L, C) erzeugt wird, wobei die Magnetresonanzmessungen der ersten Magnetresonanzdaten ($v_i$) von zueinander komplementären Folgen (10) unmittelbar nacheinander durchgeführt werden, oder

- Bereitstellen gemäß a) und b) erfasster erster Magnetresonanzdaten ($v_i$), und
- Bereitstellen einer zweiwertigen Bestimmungsmatrix, wobei eine Zeile der Bestimmungsmatrix eine Folge (10) von markierten und/oder nicht-markierten Fluidboli (L, C) repräsentiert und unterschiedliche Zeilen der Bestimmungsmatrix unterschiedlichen Magnetresonanzmessungen der ersten Magnetresonanz-Daten ($v_i$) zugrunde gelegt werden, wobei die Bestimmungsmatrix durch eine Kombination einer Walsh-sortierten Hadamard-Matrix mit einer komplementären Walsh-sortierten Hadamard-Matrix gebildet ist, wobei die gebildete Matrix wechselweise die Zeilen der Walsh-sortierten Hadamard-Matrix und der komplementären Walsh-sortierten Hadamard-Matrix aufweist,
- Auswerten der bereitgestellten ersten Magnetresonanzdaten ($v_i$) unter Berücksichtigung der Bestimmungsmatrix, wobei das Auswerten umfasst, basierend auf einer Zusammenfügung der in den verschiedenen Magnetresonanzmessungen gemessenen ersten Magnetresonanzdaten ($v_i$)

i) zweite Einzelfluidbolus-Magnetresonanzdaten ($b_i$; 41-48) zu bestimmen, die jeweils für einen zu einem bestimmten ersten Zeitpunkt erzeugten und zum zweiten Bereich geströmten einzelnen Fluidbolus (L, C) indikativ sind, und
ii) zweite Kombinationsfluidbolus-Magnetresonanzdaten ($K_i$; 30-40) zu bestimmen, die jeweils für einen Kombinations-Fluidbolus und einen zeitlichen Abstand (TI) zwischen einem der ersten Zeitpunkte und dem zweiten Zeitpunkt indikativ sind, wobei der Kombinations-Fluidbolus aus mehreren zu verschiedenen ersten Zeitpunkten markierten/nichtmarkieren und zum zweiten Bereich geströmten Fluidboli (L, C) zusammengesetzt ist, wobei

- die ersten Magnetresonanzdaten ($v_i$) in einer Reihenfolge erfasst werden, in der, bereits bevor sämtliche erste Magnetresonanzdaten ($v_i$) gemessen worden sind, die zur Bestimmung der zweiten Einzelfluidbolus-Magnetresonanzdaten ($b_i$; 41-48) benötigt werden, zweite Kombinationsfluidbolus-Magnetresonanzdaten ($K_i$; 30-40) bestimmt werden können, **dadurch gekennzeichnet, dass** die Walsh-sortierte Hadamard-Matrix und die komplementäre Walsh-sortierte Hadamard-Matrix jeweils einen Grad $2^k$ mit $k \geq 2$ aufweisen, und
- die ersten Magnetresonanzdaten ($v_i$) in einer Reihenfolge ausgewertet werden, die es ermöglicht, dass, bereits bevor sämtliche erste Magnetresonanzdaten ($v_i$) gemessen worden sind, die zur Bestimmung der zweiten Einzelfluidbolus-Magnetresonanzdaten ($b_i$; 41-48) benötigt werden, zweite Kombinationsfluidbolus-Magnetresonanzdaten ($K_i$; 30-40) bestimmt werden können, wobei für die Bestimmung von zweiten Magnetresonanzdaten für einen Kombinationsfluidbolus weniger erste Magnetresonanzdaten verwendet werden als für die Bestimmung von zweiten Magnetresonanzdaten für einen Einzelfluidbolus.

**5.** Verfahren nach Anspruch 4, bei dem die ersten Magnetresonanzdaten ($v_i$) in einer Reihenfolge ausgewertet werden, bei der die Anzahl von Wechseln zwischen markierten und nicht-markierten Fluidboli (L, C) in den zu den jeweiligen, in die Auswertung einfließenden ersten Magnetresonanzdaten ($v_i$) gehörenden Fluidboli-Folgen (10) zunimmt.

**6.** Computerprogramm zum Auswerten von diagnostisch verwendbaren Magnetresonanzdaten ($K_i$, $b_i$) eines strömenden Fluids, **dadurch gekennzeichnet, dass** das Computerprogramm Programmcodemittel aufweist, die dazu führen, dass das System nach Anspruch 1 das Verfahren nach Anspruch 4 durchführt, wenn das Computerprogramm auf einem Computer, der das System steuert, ausgeführt wird.

## Claims

**1.** System (1, 20) for evaluating diagnostically usable magnetic resonance data ($K_i$, $b_i$) of a flowing fluid, wherein the system (1, 20) comprises

- a data collection unit (5) configured to perform a plurality of magnetic resonance measurements, wherein in each magnetic resonance measurement the data collection unit (5) measures first magnetic resonance data ($v_i$), in that

in each magnetic resonance measurement

a) at different first points in time in a first region, the fluid is either magnetically labeled or not labeled such that a flowing sequence (10) of labeled (L) and/or unlabeled (C) fluid boli is generated, and

b) at a second point in time in a second region, to which the fluid, starting at the first region, flows, the first magnetic resonance data ($v_i$), which are indicative of the generated sequence (10) of labeled and/or unlabeled fluid boli (L, C), are measured, wherein the data collection unit (5) is configured to generate, for each sequence (10) of labeled and/or unlabeled fluid boli (L, C), a complementary sequence of labeled and/or unlabeled fluid boli (L, C), wherein the magnetic resonance measurements of the first magnetic resonance data ($v_i$) of sequences (10) complementary to each other are carried out one immediately after the other, and

to provide the first magnetic resonance data ($v_i$) thus measured, or
- a data provision unit (21) configured to provide first magnetic resonance data ($v_i$) measured according to a) and b), and
- a matrix provision unit (6) for providing a two-valued determination matrix, wherein a row of the determination matrix represents a sequence (10) of labeled and/or unlabeled fluid boli (L, C), and different rows of the determination matrix form the basis for different magnetic resonance measurements of the first magnetic resonance data ($v_i$),
wherein the determination matrix is formed by a combination of a Walsh-sorted Hadamard matrix with a complementary Walsh-sorted Hadamard matrix, wherein the resulting matrix alternately comprises the rows of the Walsh-sorted Hadamard matrix and the complementary Walsh-sorted Hadamard matrix,
- an evaluation unit (7, 22) configured to, based on a combination of the first magnetic resonance data ($v_i$) measured in the different magnetic resonance measurements, taking into account the determination matrix,

i) determine second individual fluid bolus magnetic resonance data ($b_i$; 41-48), each of which are indicative of an individual fluid bolus (L, C) generated at a certain first point in time and flowed to the second region, based on a combination of the first magnetic resonance data ($v_i$) measured in the different magnetic resonance measurements, and

ii) determine second combination fluid bolus magnetic resonance data ($K_i$; 30-40), each of which are indicative of a combination fluid bolus and a time interval (TI) between one of the first points in time and the second point in time, wherein the combination fluid bolus is combined from a plurality of fluid boli (L, C) labeled/unlabeled at different first points in time and flowed to the second region,

wherein
- the data collection unit (5) is configured to measure the first magnetic resonance data ($v_i$) in an order that allows second combination fluid bolus magnetic resonance data ($K_i$; 30-40) to be determined in the evaluation unit (7, 22) even before all first magnetic resonance data ($v_i$) required to determine the second individual fluid bolus magnetic resonance data ($b_i$; 41-48) have been measured, **characterized in that** the Walsh-sorted Hadamard matrix and the complementary Walsh-sorted Hadamard matrix each are of order $2^k$ with $k \geq 2$, and
- the evaluation unit (7, 22) is configured to evaluate the first magnetic resonance data ($v_i$) in an order that allows second combination fluid bolus magnetic resonance data ($K_i$; 30-40) to be determined even before all first magnetic resonance data ($v_i$) required to determine the second individual fluid bolus magnetic resonance data ($b_i$; 41-48) have been measured, wherein fewer first magnetic resonance data are used to determine second magnetic resonance data for a combination fluid bolus than are used to determine second magnetic resonance data for an individual fluid bolus.

2. System (1, 20) according to claim 1, **characterized in that** the evaluation unit (7, 22) is configured to evaluate the first magnetic resonance data ($v_i$) in an order in which the number of changes between labeled and unlabeled fluid boli (L, C) in the fluid boli sequences (10) belonging to the respective first magnetic resonance data entering into the evaluation increases.

3. System (1, 20) according to claim 1 or 2, **characterized in that** the rows of the Walsh-sorted Hadamard matrix represent a first group of sequences (10) of labeled/unlabeled fluid boli (L, C), wherein different rows of the Walsh-sorted Hadamard matrix form the basis for different magnetic resonance measurements of the first magnetic resonance data ($v_i$), and the rows of the complementary Walsh-sorted Hadamard matrix represent a second group of sequences (10) of labeled/unlabeled fluid boli (C, L), which are complementary to the sequences of fluid boli of the first group, wherein different rows of the complementary Walsh-sorted Hadamard matrix form the basis for different magnetic resonance measurements of the first magnetic resonance data ($v_i$), wherein the evaluation unit (7, 22) is further configured

- to form a first vector, wherein the vector elements of the first vector are each formed by a magnetic resonance value of the first magnetic resonance data ($v_i$), and different vector elements of the first vector correspond to different magnetic resonance measurements of the first magnetic resonance data ($v_i$), which in turn correspond to different fluid boli sequences of the first group,
- to form a second vector, wherein a vector element of the second vector is formed by a magnetic resonance value of the first magnetic resonance data ($v_i$), and different vector elements of the second vector correspond to different magnetic resonance measurements of the first magnetic resonance data ($v_i$), which in turn correspond to different fluid boli sequences of the second group,
- to invert the Walsh-sorted Hadamard matrix and the complementary Walsh-sorted Hadamard matrix, and
- to determine the second magnetic resonance data by applying the inverted Walsh-sorted Hadamard matrix to the first vector and the inverted complementary Walsh-sorted Hadamard matrix to the second vector.

4. Method for evaluating diagnostically usable magnetic resonance data of a flowing fluid, wherein the method comprises:

- collecting and providing first magnetic resonance data ($v_i$), wherein a plurality of magnetic resonance measurements are carried out, wherein in each magnetic resonance measurement first magnetic resonance data ($v_i$) are measured, in that in each magnetic resonance measurement

a) at different first points in time in a first region of the fluid, the fluid is either magnetically labeled or not labeled such that a flowing sequence (10) of labeled (L) and/or unlabeled (C) fluid boli is generated, and
b) at a second point in time in a second region, to which the fluid, starting at the first region, flows, the first magnetic resonance data ($v_i$), which are indicative of the generated sequence (10) of labeled and/or unlabeled fluid boli (L, C), are measured, wherein for each sequence (10) of labeled and/or unlabeled fluid boli (L, C), a complementary sequence of labeled and/or unlabeled fluid boli (L, C) is generated, wherein the magnetic resonance measurements of the first magnetic resonance data ($v_i$) of sequences (10) complementary to each other are carried out one immediately after the other, or

- providing first magnetic resonance data ($v_i$) measured according to a) and b), and
- providing a two-valued determination matrix, wherein a row of the determination matrix represents a sequence (10) of labeled and/or unlabeled fluid boli (L, C), and different rows of the determination matrix form the basis for different magnetic resonance measurements of the first magnetic resonance data ($v_i$),
wherein the determination matrix is formed by a combination of a Walsh-sorted Hadamard matrix with a complementary Walsh-sorted Hadamard matrix, wherein the resulting matrix alternately comprises the rows of the Walsh-sorted Hadamard matrix and the complementary Walsh-sorted Hadamard matrix,
- evaluating the provided first magnetic resonance data ($v_i$), taking into account the determination matrix, wherein the evaluating comprises, based on a combination of the first magnetic resonance data ($v_i$) measured in the different magnetic resonance measurements

i) to determine second individual fluid bolus magnetic resonance data ($b_i$; 41-48), each of which are indicative of an individual fluid bolus (L, C) generated at a certain first point in time and flowed to the second region, and
ii) to determine second combination fluid bolus magnetic resonance data ($K_i$; 30-40), each of which are indicative of a combination fluid bolus and a time interval (TI) between one of the first points in time and the second point in time, wherein the combination fluid bolus is combined from a plurality of fluid boli (L, C) labeled/unlabeled at different first points in time and flowed to the second region,

wherein
- the first magnetic resonance data ($v_i$) are collected in an order in which, even before all first magnetic resonance data ($v_i$) required to determine the second individual fluid bolus magnetic resonance data ($b_i$; 41-48) have been measured, second combination fluid bolus magnetic resonance data ($K_i$; 30-40) can be determined, **characterized in that** the Walsh-sorted Hadamard matrix and the complementary Walsh-sorted Hadamard matrix each are of order $2^k$ with $k \geq 2$, and
- the first magnetic resonance data ($v_i$) are evaluated in an order that allows second combination fluid bolus magnetic resonance data ($K_i$; 30-40) to be determined even before all first magnetic resonance data ($v_i$) required to determine the second individual fluid bolus magnetic resonance data ($b_i$; 41-48) have been measured, wherein fewer first magnetic resonance data are used to determine second magnetic resonance data for a combination fluid bolus than are used to determine second magnetic resonance data for an individual fluid bolus.

5. Method according to claim 4, wherein the first magnetic resonance data ($v_i$) are evaluated in an order in which the number of changes between labeled and unlabeled fluid boli (L, C) in the fluid boli sequences (10) belonging to the respective first magnetic resonance data entering into the evaluation increases.

6. Computer program for evaluating diagnostically usable magnetic resonance data ($K_i$, $b_i$) of a flowing fluid, **characterized in that** the computer program comprises program code means which cause the system according to claim 1 to carry out the method according to claim 4 when the computer program is executed on a computer which controls the system.

**Revendications**

1. Système (1, 20) d'évaluation de données de résonance magnétique ($K_i$, $b_i$) utilisables pour le diagnostic d'un fluide en écoulement, dans lequel le système (1, 20) présente :

- une unité d'acquisition de données (5) qui est adaptée pour effectuer plusieurs mesures par résonance magnétique et mesure à cet effet dans chaque mesure par résonance magnétique des premières données par résonance magnétique ($v_i$), du fait que dans chaque mesure par résonance magnétique

a) à différents premiers moments dans une première zone, le fluide est soit marqué magnétiquement soit non marqué, de sorte qu'une séquence (10) d'écoulement de bolus de fluide marqués (L) et/ou non marqués (C) se forme, et
b) à un deuxième moment dans une deuxième zone où le fluide s'écoule à partir de la première zone, les premières données de résonance magnétique ($v_i$), qui sont indicatives de la séquence (10) générée de bolus de fluide marqués et/ou non marqués (L, C), sont mesurées, dans lequel l'unité d'acquisition de données (5) est adaptée, pour chaque séquence (10) de bolus de fluide marqués et/ou non marqués (L, C), pour générer une séquence complémentaire de bolus de fluide marqués et/ou non marqués (L, C), dans lequel les mesures de résonance magnétique des premières données de résonance magnétique ($v_i$) de séquences (10) complémentaires les unes aux autres sont effectuées immédiatement l'une après l'autre, et

pour fournir les premières données de résonance magnétique ($v_i$) ainsi mesurées, ou
- une unité de fourniture de données (21), qui est adaptée pour fournir des premières données de résonance magnétique ($v_i$) mesurées selon a) et b), et
- une unité de fourniture de matrice (6) pour fournir une matrice de détermination bivalente, dans lequel une ligne de la matrice de détermination représente une séquence (10) de bolus de fluide marqués et/ou non marqués (L, C) et différentes lignes de la matrice de détermination sont basées sur différentes mesures de résonance magnétique des premières données de résonance magnétique ($v_i$),
dans lequel la matrice de détermination est formée par une combinaison d'une matrice de Hadamard triée par Walsh avec une matrice de Hadamard triée par Walsh complémentaire, dans lequel la matrice formée présente alternativement les lignes de la matrice de Hadamard triée par Walsh et de la matrice de Hadamard triée par Walsh complémentaire,
- une unité d'évaluation (7, 22) qui est adaptée, sur la base d'une réunion des premières données de résonance magnétique ($v_i$) mesurées dans les différentes mesures de résonance magnétique en tenant compte de la matrice de détermination,

i) pour déterminer des deuxièmes données de résonance magnétique de bolus de fluide unique ($b_i$ ; 41-48), qui sont respectivement indicatives d'un bolus de fluide unique (L, C) généré à un premier moment donné et écoulé vers la deuxième zone, sur la base d'une réunion des premières données de résonance magnétique ($v_i$) mesurées dans les différentes mesures de résonance magnétique, et
ii) pour déterminer des deuxièmes données de résonance magnétique de bolus de fluide combiné ($K_i$ ; 30-40), qui sont respectivement indicatives d'un bolus de fluide combiné et d'un écart temporel (TI) entre l'un des premiers moments et le deuxième moment, dans lequel le bolus de fluide combiné est composé de plusieurs bolus de fluide marqués/non marqués (L, C) à différents premiers moments et écoulés vers la deuxième zone, dans lequel

- l'unité d'acquisition de données (5) est adaptée pour mesurer les premières données de résonance magnétique ($v_i$) dans un ordre qui permet, avant même que toutes les premières données de résonance magnétique ($v_i$) qui sont nécessaires à la détermination des deuxièmes données de résonance magnétique de bolus de fluide unique ($b_i$ ; 41-48) aient été mesurées, que les deuxièmes données de résonance magnétique de bolus de fluide combiné ($K_i$; 30-40) puissent être déterminées dans l'unité d'évaluation (7, 22), **caractérisé en ce que** la matrice de Hadamard triée par Walsh et la matrice de Hadamard triée par Walsh complémentaire présentent chacune un degré $2^k$ avec $k \geq 2$, et

- l'unité d'évaluation (7, 22) est adaptée pour évaluer les premières données de résonance magnétique ($v_i$) dans un ordre qui permet, avant même que toutes les premières données de résonance magnétique ($v_i$) qui sont nécessaires à la détermination des deuxièmes données de résonance magnétique de bolus de fluide unique ($b_i$ ; 41-48) aient été mesurées, que des deuxièmes données de résonance magnétique de bolus de fluide combiné ($K_i$ ; 30-40) puissent être déterminées, dans lequel moins de premières données de résonance magnétique sont utilisées pour la détermination de deuxièmes données de résonance magnétique pour un bolus de fluide combiné que pour la détermination de deuxièmes données de résonance magnétique pour un bolus de fluide unique.

2. Système (1, 20) selon la revendication 1, **caractérisé en ce que** l'unité d'évaluation (7, 22) est adaptée pour évaluer les premières données de résonance magnétique ($v_i$) dans un ordre dans lequel le nombre de changements entre les bolus de fluide marqués et non marqués (L, C) dans les premières séquences de bolus de fluide (10) faisant partie des premières données de résonance magnétique ($v_i$) respectives entrant dans l'évaluation augmente.

3. Système (1, 20) selon la revendication 1 ou 2, **caractérisé en ce que** les lignes de la matrice de Hadamard triée par Walsh représentent un premier groupe de séquences (10) de bolus de fluide marqués/non marqués (L, C), dans lequel différentes lignes de la matrice de Hadamard triée par Walsh sont basées sur différentes mesures par résonance magnétique des premières données de résonance magnétique ($v_i$), et les lignes de la matrice de Hadamard triée par Walsh complémentaire représentent un deuxième groupe de séquences (10) de bolus de fluide marqués/non marqués (C, L), qui sont complémentaires des séquences de bolus de fluide du premier groupe, dans lequel différentes lignes de la matrice de Hadamard triée par Walsh complémentaire sont basées sur différentes mesures par résonance magnétique des premières données de résonance magnétique ($v_i$), dans lequel l'unité d'évaluation (7, 22) est en outre adaptée

- pour former un premier vecteur dont les éléments vectoriels sont formés chacun par une valeur de résonance magnétique des premières données de résonance magnétique ($v_i$) et différents éléments vectoriels du premier vecteur correspondent à différentes mesures de résonance magnétique des premières données de résonance magnétique ($v_i$), qui correspondent à leur tour à différentes séquences de bolus de fluide du premier groupe,

- pour former un deuxième vecteur, dans lequel un élément vectoriel du deuxième vecteur est formé par une valeur de résonance magnétique des premières données de résonance magnétique ($v_i$) et différents éléments vectoriels du deuxième vecteur correspondent à différentes mesures de résonance magnétique des premières données de résonance magnétique ($v_i$) qui correspondent à leur tour à différentes séquences de bolus de fluide du deuxième groupe,

- pour inverser la matrice de Hadamard triée par Walsh et la matrice de Hadamard triée par Walsh complémentaire, et

- pour déterminer les deuxièmes données de résonance magnétique en appliquant la matrice de Hadamard triée par Walsh inversée au premier vecteur et la matrice de Hadamard triée par Walsh complémentaire inversée au deuxième vecteur.

4. Procédé d'évaluation de données de résonance magnétique d'un fluide en écoulement utilisables pour le diagnostic, dans lequel le procédé présente :

- l'acquisition et la fourniture de premières données de résonance magnétique ($v_i$), dans lequel plusieurs mesures de résonance magnétique sont effectuées et ce faisant les premières données de résonance magnétique ($v_i$) sont mesurées dans chaque mesure de résonance magnétique, du fait que dans chaque mesure de résonance magnétique

a) à différents premiers moments dans une première zone du fluide, celui-ci est soit marqué magnétiquement soit non marqué de sorte qu'une séquence d'écoulement (10) de bolus de fluide marqués (L) et/ou non marqués (C) est générée, et

b) à un deuxième moment, à un deuxième endroit où le fluide s'écoule à partir de la première zone, les premières données de résonance magnétique ($v_i$) qui sont indicatives de la séquence (10) générée de bolus de fluide marqués et/ou non marqués (L, C) sont mesurées, dans lequel, pour chaque séquence (10) de bolus de fluide marqués et/ou non marqués (L, C), une séquence complémentaire de bolus de fluide marqués et/ou non marqués (L, C) est générée, dans lequel les mesures de résonance magnétique des premières données de résonance magnétique ($v_i$) de séquences (10) complémentaires les unes aux autres sont effectuées immédiatement l'une après l'autre, ou

- la fourniture de premières données de résonance magnétique ($v_i$) acquises selon a) et b), et
- la fourniture d'une matrice de détermination bivalente, dans lequel une ligne de la matrice de détermination représente une séquence (10) de bolus de fluide marqués et/ou non marqués (L, C) et différentes lignes de la matrice de détermination sont basées sur différentes mesures par résonance magnétique des premières données de résonance magnétique ($v_i$),
dans lequel la matrice de détermination est formée par une combinaison d'une matrice de Hadamard triée par Walsh avec une matrice de Hadamard triée par Walsh complémentaire, dans lequel la matrice formée présente alternativement les lignes de la matrice de Hadamard triée par Walsh et de la matrice de Hadamard triée par Walsh complémentaire,
- l'évaluation des premières données de résonance magnétique ($v_i$) fournies en tenant compte de la matrice de détermination, dans lequel l'évaluation comprend, sur la base d'une réunion des premières données de résonance magnétique ($v_i$) mesurées dans les différentes mesures de résonance magnétique

i) pour déterminer des deuxièmes données de résonance magnétique de bolus de fluide unique ($b_i$ ; 41-48) qui sont respectivement indicatives d'un bolus de fluide unique (L, C) généré à un premier moment donné et écoulé vers la deuxième zone, et
ii) pour déterminer des deuxièmes données de résonance magnétique de bolus de fluide combiné ($K_i$ ; 30-40) qui sont respectivement indicatives d'un bolus de fluide combiné et d'un écart temporel (TI) entre l'un des premiers moments et le deuxième moment, dans lequel le bolus de fluide combiné est composé de plusieurs bolus de fluide marqués/non marqués à différents premiers moments et écoulés vers la deuxième zone (L, C), dans lequel

- les premières données de résonance magnétique ($v_i$) sont acquises dans un ordre dans lequel, avant même que toutes les premières données de résonance magnétique ($v_i$) qui sont nécessaires à la détermination des deuxièmes données de résonance magnétique de bolus de fluide unique ($b_i$ ; 41-48) aient été mesurées, les deuxièmes données de résonance magnétique de bolus de fluide combiné ($K_i$ ; 30-40) peuvent être déterminées, **caractérisé en ce que**
la matrice de Hadamard triée par Walsh et la matrice de Hadamard triée par Walsh complémentaire présentent chacune un degré $2^k$ avec $k \geq 2$, et
- les premières données de résonance magnétique ($v_i$) sont évaluées dans un ordre qui permet, avant même que toutes les premières données de résonance magnétique ($v_i$) qui sont nécessaires à la détermination des deuxièmes données de résonance magnétique de bolus de fluide unique ($b_i$ ; 41-48) aient été mesurées, que les deuxièmes données de résonance magnétique de bolus de fluide combiné ($K_i$ ; 30-40) puissent être déterminées, dans lequel moins de premières données de résonance magnétique sont utilisées pour la détermination de deuxièmes données de résonance magnétique pour un bolus de fluide combiné que pour la détermination de deuxièmes données de résonance magnétique pour un bolus de fluide unique.

5. Procédé selon la revendication 4, selon lequel les premières données de résonance magnétique ($v_i$) sont évaluées dans un ordre dans lequel le nombre de changements entre les bolus de fluide marqués et non marqués (L, C) dans les séquences de bolus de fluide (10) appartenant aux premières données de résonance magnétique ($v_i$) respectives entrant dans l'évaluation augmente.

6. Programme informatique pour l'évaluation de données de résonance magnétique ($K_i$, bi) d'un fluide en écoulement utilisables pour le diagnostic, **caractérisé en ce que** le programme informatique présente des moyens de codage de programme qui ont pour effet que le système selon la revendication 1 met en œuvre le procédé selon la revendication 4 lorsque le programme informatique est exécuté sur un ordinateur qui commande le système.

FIG. 1

FIG. 2

EP 3 123 193 B1

FIG. 3

FIG. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8260396 B2, Guenther
- US 8610433 B2

**In der Beschreibung aufgeführte Nicht-Patentliteratur**

- **W. TEEUWISSE et al.** Time-Encoded pseudoContinuous Arterial Spin Labeling: Basic Properties and Timing Strategies for Human Applications. *Magnetic Resonance in Medicine*, 2014, vol. 72, 1712-1722
- **BUXTON, R.B. et al.** A general kinetic model for quantitative perfusion imaging with arterial spin labeling. *Magnetic Resonance in Medicine*, 1998, vol. 40 (3), 383-96
- **PAYNE, G.S.** ; **LEACH, M.O.** Implementation and evaluation of frequency offset corrected inversion (FOCI) pulses on a clinical MR system. *Magnetic resonance in medicine*, 1997, vol. 38 (5), 828-33
- **DAI, W. et al.** Continuous flow-driven inversion for arterial spin labeling using pulsed radio frequency and gradient fields. *Magnetic resonance in medicine*, 2008, vol. 60 (6), 1488-97
- **GADIAN, D.G.** *NMR & Its Applications to Living Systems 2 Sub.*, 1996
- **BERNSTEIN, M.** ; **KING, K.** ; **ZHOU, X.** Handbook of MRI pulse sequences. Academic Press, 2004
- **WILLIAMS, D.S. et al.** Magnetic resonance imaging of perfusion using spin inversion of arterial water. *Proceedings of the National Academy of Sciences of the United States of America*, 1992, vol. 89 (1), 212-6
- **DETRE, J.** ; **LEIGH, J.** ; **WILLIAMS, D.** Perfusion imaging. *Magnetic Resonance in Medicine*, 1996, vol. 35 (1), 70-9
- **GÜNTHER, M.** Habilitationsschrift. Ruprechts-Karl-Universität, 2007
- **GÜNTHER, M.** ; **OSHIO, K.** ; **FEINBERG, D.A.** Single-shot 3D imaging techniques improve arterial spin labeling perfusion measurements. *Magnetic resonance in medicine*, 2005, vol. 54 (2), 491-498
- **DAI, W.** ; **SHANKARANARAYANAN, A.** ; **ALSOP, D.C.** Volumetric measurement of perfusion and arterial transit delay using hadamard encoded continuous arterial spin labeling. *Magnetic resonance in medicine*, 2013, vol. 69 (4), 1014-22
- **TEEUWISSE, W.M. et al.** Time-encoded pseudo-continuous arterial spin labeling: Basic properties and timing strategies for human applications. *Magnetic resonance in medicine*, 2014